(19) Europäisches Patentamt
European Patent Office
Office européen des brevets

(11) **EP 2 564 760 A1**

(12) # EUROPEAN PATENT APPLICATION

(43) Date of publication:
**06.03.2013 Bulletin 2013/10**

(51) Int Cl.:
***A61B 1/06*** *(2006.01)*

(21) Application number: **12177855.9**

(22) Date of filing: **25.07.2012**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br><br>(30) Priority: **29.08.2011 JP 2011186345**<br> **13.09.2011 JP 2011199402**<br><br>(71) Applicant: **Fujifilm Corporation**<br>**Minato-ku**<br>**Tokyo (JP)** | (72) Inventors:<br>• **YAMAMOTO, Hiroaki**<br> **Kanagawa (JP)**<br>• **SAITO, Takaaki**<br> **Kanagawa (JP)**<br>• **YAMAGUCHI, Hiroshi**<br> **Kanagawa (JP)**<br><br>(74) Representative: **Klunker . Schmitt-Nilson . Hirsch**<br>**Patentanwälte**<br>**Destouchesstrasse 68**<br>**80796 München (DE)** |

(54) **Endoscopic diagnosis system**

(57) An endoscopic diagnosis system includes a light source unit (12) for emitting green and red light, blue and green light, or white light (LD1) and excitation light (LD2) having different central wavelengths for causing an autofluorescent substance to emit autofluorescence, an imaging unit (16) for receiving reflected light from the observation region to acquire a reflected light image when the observation region is illuminated with the illumination light emitted, and receiving autofluorescence that is emitted from the autofluorescent substance, absorbed by blood, and decreases according to the amount of the blood to acquire an autofluorescence image when the observation region is irradiated with the excitation light emitted, and an image correction unit (68) for correcting the autofluorescence image based on the reflected light image.

## FIG.18

| | NORMAL REGION | LESION | ASSIGNMENT |
|---|---|---|---|
| FAD:G−CHANNEL FLUORESCENCE IMAGE WITH 445−nm EXCITATION LIGHT | BRIGHT ↗ | DARK G ↘ | G |
| PORPHYRIN: R−CHANNEL FLUORESCENCE IMAGE WITH 405−nm EXCITATION LIGHT | DARK G ↘ | BRIGHT ↗ | R,B |

EP 2 564 760 A1

**Description**

BACKGROUND OF THE INVENTION

The present invention relates to an endoscopic diagnosis system for acquiring an autofluorescence image by imaging autofluorescence emitted from an autofluorescent substance contained in a subject's observation region (organism).

There is conventionally used an endoscope device in which normal light (white light) emitted from a light source device is guided to the tip of an endoscope to illuminate an observation region of a subject, and the reflected light is imaged to acquire a normal light image (white light image) for normal light observation (white light observation). In recent years, there is used an endoscope device in which a region of a subject under observation is irradiated with excitation light (special light) used for observation of autofluorescence, and autofluorescence emitted from an autofluorescent substance is imaged to acquire an autofluorescence image (special light image) for autofluorescence observation (special light observation) in addition to normal light observation.

Among endoscope devices for autofluorescence observation are those described in JP 2009-95683 A and JP 2008-43383 A.

The endoscope device described in JP 2009-95683 comprises two excitation light and an etalon disposed before a sensor and having a variable transmitted wavelength range. The endoscope device performs image computation from fluorescence images acquired in wavelength regions of the excitation light adapted to the fluorescence wavelength of a target substance, extracts images of collagen elastin, porphyrin, and the fluorescence probe, and assigns the image of collagen elastin to the R channel, porphyrin to the G channel, and the fluorescence probe to the B channel, thereby achieving pseudo-color display.

With the device described in JP 2009-95683 A, as illustrated in Figs. 27 and 28, the subject is first irradiated with excitation light A having a wavelength of 405 nm in a first and a second frame. In the first frame, an image signal D1 containing an autofluorescence component having a wavelength region a1 emitted from collagen elastin and an autofluorescence component having a wavelength region a2 emitted from porphyrin is acquired; in the second frame, an image signal D2 containing an autofluorescence component having a wavelength region a1 is acquired. In a third frame to follow, the subject is irradiated with excitation light B having a wavelength of 660 nm to acquire an image signal D3 containing a fluorescence component having a wavelength region a3 emitted from the fluorescence probe.

Upon acquisition of the image signals D1 to D3, the image signal D2 is subtracted from the image signal D1 to produce an image signal E1 containing only a fluorescence component having a wavelength region a2. The image signal D2 is assigned to the R channel, the image signal E1 to the G channel, and the image signal D3 to the B channel to display a fluorescence image in pseudo color. Thus, the fluorescence image shows portions of a body tissue in different colors according to their states: a normal region is shown in yellow, an inflamed region in gray, and a lesion in magenta, as shown in Table 1 below.

(Table 1)

|  | Normal region (yellow) | Inflamed region (gray) | Lesion (magenta) | Assignment |
|---|---|---|---|---|
| Collagen elastin: Excitation light A (405 nm) Image signal D2 | Bright | Dark | Dark | R |
| Porphyrin: Excitation light A (405 nm) Image signal E1 | Bright | Dark | Bright | G |
| Fluorescence probe: Excitation light B (660 nm) Image signal D3 | Dark | Dark | Bright | B |

JP 2008-43383 A describes a method of obtaining an autofluorescence image comprising sequentially irradiating a body tissue with a plurality of excitation light having a wavelength of 450 nm or less and cutting off the excitation light with an excitation light cutoff filter provided on the light receiving surface of a sensor. Objects observed with fluorescence by the method described in JP 2008-43383 A include autofluorescence emitted from, for example, NADH and collagen and tumor-seeking substances such as porphyrin administered for PDD or photodynamic diagnosis.

SUMMARY OF THE INVENTION

Blood absorbs autofluorescence, so that the intensity of autofluorescence signal decreases as the amount of blood increases in an inflamed region. The method described in JP 2009-95683 A uses this fact to determine a darkened region as inflamed region.

However, the method of JP 2009-95683 A is incapable of distinguishing an inflamed region from a region containing a large quantity of blood such as a thick blood vessel, which is not an inflamed region, and therefore one cannot know whether it is an inflamed region or a large quantity of blood that is under observation. Also in a lesion, the amount of blood increases, and the signal intensity hence decreases accordingly. The method of JP 2009-95683 A had a problem that the fluorescence intensity must be increased with a fluorescence probe to avoid the weakening of the signal; otherwise sufficient use of fluorescence emitted from an autofluorescent substance was impossible.

Part of the excitation light and fluorescence emitted from a body tissue is absorbed by blood contained in the body tissue according to the amount of blood. Therefore, as in JP 2008-43383 A, when autofluorescence is simply imaged and observed, determining whether increase and decrease in intensity of the observed auto fluoresce is due to the magnitude of the amount of an autofluorescent substance emitting the autofluorescence or due to absorption varying with the amount of blood, causing uncertainty in diagnosis.

An object of the present invention is to provide an endoscopic diagnosis system capable of eliminating the effects of light absorption by blood without the need to use a fluorescence probe and obtaining an autofluorescence image that is in accordance with the increase and decrease in the amount of an autofluorescent substance without depending on the amount of blood in a body tissue.

In order to achieve the above object, the present invention provides an endoscopic diagnosis system comprising:

a light source unit for emitting illumination light including at least green and red light or blue and green light or white light, and one or more kinds of excitation light having different central wavelengths for causing at least one autofluorescent substance contained in a subject's observation region to emit one or more kinds of autofluorescence;

an imaging unit for receiving reflected light of the illumination light from the subject's observation region to acquire a reflected light image when the subject's observation region is illuminated with the illumination light emitted from the light source unit, and receiving autofluorescence that is emitted from the at least one autofluorescent substance contained in the subject's observation region, absorbed by blood, and decreases according to an amount of the blood to acquire an autofluorescence image when the subject's observation region is irradiated with the excitation light emitted from the light source unit; and

an image correction unit for correcting the autofluorescence image based on the reflected light image.

Also, the present invention provides the endoscopic diagnosis system according to above,

wherein the light source unit is adapted to emit the white light as the illumination light and first excitation light as the excitation light,

wherein the image correction unit has correction coefficients for correcting a signal intensity of the autofluorescence image for decrease occurring in accordance with the amount of the blood as the autofluorescence emitted from the at least one autofluorescent substance contained in the subject's observation region is absorbed by the blood, and

wherein the image correction unit is adapted to obtain a correction coefficient corresponding to a reflectance of the reflected light image from among correction coefficients and use the obtained correction coefficient to correct the signal intensity of the autofluorescence image.

Also, the present invention provides the endoscopic diagnosis system according to above,

wherein the light source unit is adapted to emit the at least green and red light or blue and green light as the illumination light, and the excitation light,

wherein the image correction unit contains information on an attenuation ratio with which the excitation light and the autofluorescence emitted from the at least one autofluorescent substance contained in the subject's observation region decrease by absorption by the blood according to the amount of the blood, and

wherein the image correction unit is adapted to obtain an attenuation ratio corresponding to the image signals of the reflected light image from the information on the attenuation ratio corresponding to the amount of the blood, and use the obtained attenuation ratio to correct the image signals of the autofluorescence image.

The present invention enables acquisition of an autofluorescence image free from the effects of light absorption by blood by correcting the autofluorescence image based on a reflected light image. For example, an autofluorescence image free from the effects of light absorption by blood may be obtained by correcting the intensities of the autofluorescence image signals using a correction coefficient corresponding to the reflectance of a reflected light image having the same wavelength band as the emission wavelength band of the autofluorescence.

Further, obtaining an attenuation ratio of excitation light and autofluorescence based on the image signals of the reflected light and correcting the image signals of the autofluorescence image using the attenuation ratio enables elimination of the effects of light absorption by blood from the autofluorescence image and acquisition of the autofluorescence image that is in accordance with the increase and decrease in the amount of an autofluorescent substance without depending on the amount of blood. Further, distinction that can be made between the increase and decrease in an autofluorescent substance and the increase and decrease in absorption by blood enhances diagnosis performance in the autofluorescence observation mode.

BRIEF DESCRIPTION OF THE DRAWINGS

Fig. 1 is an external view of an embodiment representing a configuration of the endoscopic diagnosis system according to a first aspect of the invention.

Fig. 2 is a block diagram representing an internal configuration of the endoscopic diagnosis system of Fig. 1.

Fig. 3 is a conceptual view illustrating a tip portion of an endoscope insertion section of the endoscopic diagnosis system of Fig. 1.

Fig. 4 is a graph of an example of light absorption intensity characteristics of autofluorescent substances.

Fig. 5 is a graph of an example of fluorescence intensities of autofluorescent substances.

Fig. 6 is a graph of an example of fluorescence intensities of autofluorescent substances.

Fig. 7 is a graph showing light absorption coefficients of oxygenated hemoglobin and reduced hemoglobin.

Fig. 8 is a graph illustrating an example of fluorescence intensity characteristics of FAD each in the presence and absence of blood.

Fig. 9 is a graph illustrating an example of relation between reflectance and fluorescence intensity on the one hand and blood concentration on the other hand.

Fig. 10 is a graph illustrating an example of relation between fluorescence intensity and reflectance.

Fig. 11 is a graph illustrating an example of correction coefficients for correcting the fluorescence intensity of autofluorescence.

Fig. 12 is a graph having spectral transmission characteristics of color filters superimposed over the graph of Fig. 5.

Fig. 13 is a graph having spectral transmission characteristics of color filters superimposed over the graph of Fig. 6.

Fig. 14 is a graph of autofluorescence's fluorescence intensity distributions obtained when a plurality of excitation light having different wavelengths are emitted simultaneously, with spectral transmission characteristics of color filters superimposed.

Fig. 15 is a graph illustrating an example of relation between blood's absorption coefficients and color filters in a normal sensor.

Fig. 16 is a conceptual view illustrating processing performed by the endoscopic diagnosis system of Fig. 1 in normal light observation mode and autofluorescence observation mode.

Fig. 17 is a conceptual view illustrating an operation of the endoscopic diagnosis system of Fig. 1.

Fig. 18 is a table showing fluorescence intensities of FAD and porphyrin in normal region and lesion.

Fig. 19 is an external view of an embodiment representing a configuration of the endoscopic diagnosis system according to a second aspect of the invention.

Fig. 20 is a block diagram of a first embodiment illustrating an internal configuration of the endoscopic diagnosis system shown in Fig. 19.

Fig. 21A is a graph illustrating a first conversion table representing a relation between amount of blood and a ratio $R2/G2$ of a red image signal to a green image signal of a normal light image; Fig. 21B is a graph illustrating a second conversion table representing a relation between amount of blood C and attenuation ratio A.

Fig. 22 is a block diagram of the first embodiment illustrating an operation of the endoscopic diagnosis system shown in Fig. 20.

Fig. 23 is a block diagram of a second embodiment illustrating an internal configuration of the endoscopic diagnosis system shown in Fig. 19.

Fig. 24A is a graph illustrating a third conversion table representing a relation between amount of blood and a ratio $G2/B2$ of a green image signal to a blue image signal of a normal light image; Fig. 24B is a graph illustrating a fourth conversion table representing a relation between amount of blood C1 and attenuation ratio A1.

Fig. 25A is a graph illustrating a fifth conversion table representing a relation between amount of blood and a ratio $R3/G2$ of a red image signal of a monochromatic light image to a green image signal of a normal light image; Fig. 25B is a graph illustrating a sixth conversion table representing a relation between amount of blood C2 and fluorescence attenuation ratio A2.

Fig. 26 is a conceptual view of an operation of the second embodiment of the endoscopic diagnosis system shown in Fig. 23.

Fig. 27 is a conceptual view of an operation of the endoscope device described in JP 2009-95683 A.

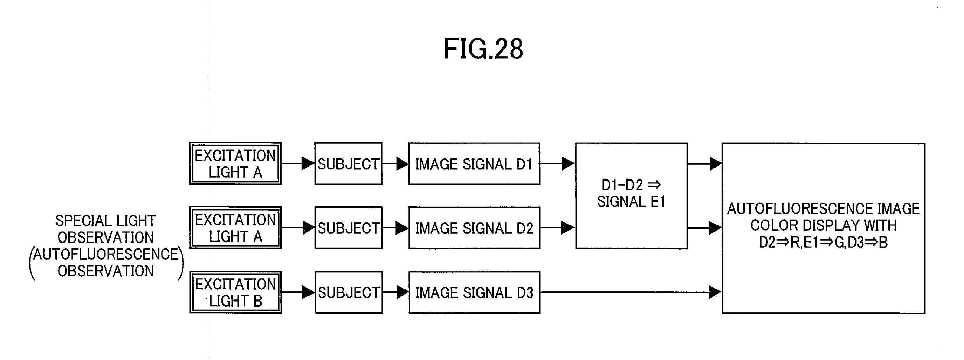

Fig. 28 is a conceptual view of a processing in an autofluorescence observation mode described in JP 2009-95683 A.

DETAILED DESCRIPTION OF THE INVENTION

The endoscopic diagnosis system according to the present invention will be described in detail below based on the preferred embodiments illustrated in the attached drawings.

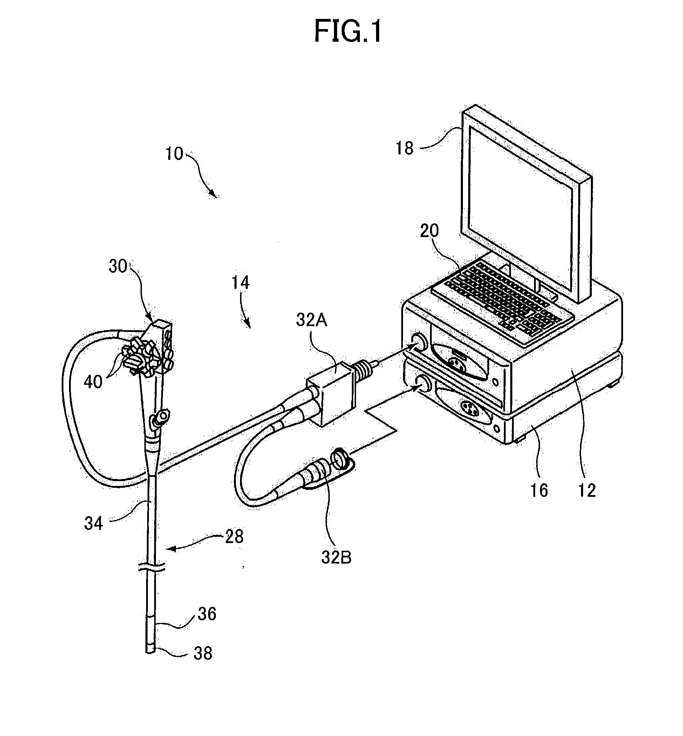

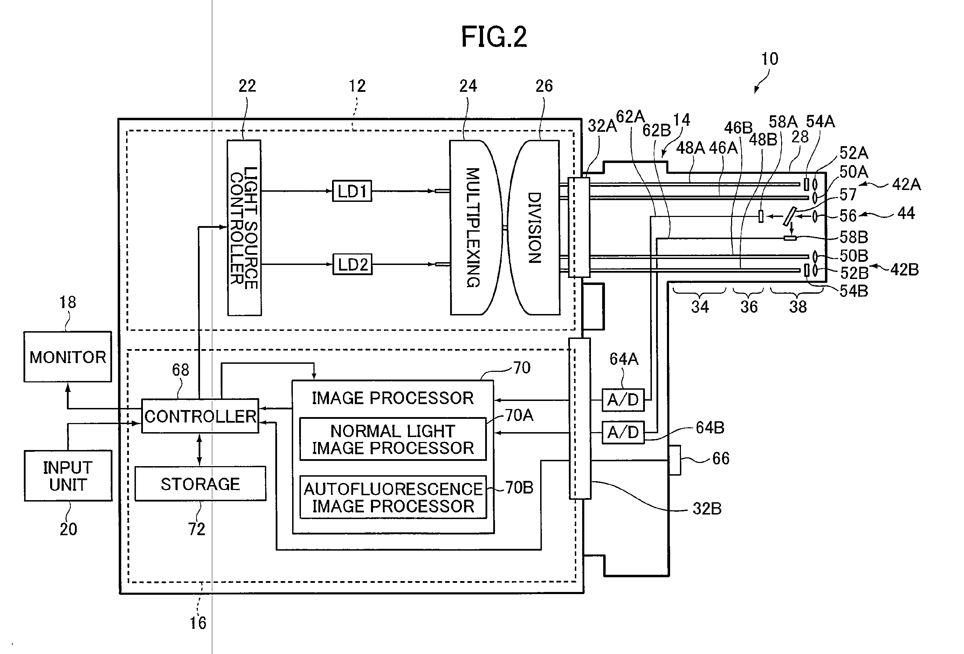

Fig. 1 is an external view of an embodiment representing a configuration of an endoscopic diagnosis system according to a first aspect of the invention; Fig. 2 is a block diagram representing an internal configuration thereof. An endoscopic diagnosis system 10 illustrated in these figures comprises a light source device 12 for emitting a plurality of light having different ranges of wavelength; an endoscope device 14 for guiding light emitted from the light source device 12 to illuminate a subject's observation region and imaging the reflected light or autofluorescence from the subject; a processor 16 for image-processing the image acquired by the endoscope device 14 and outputting an endoscopic image; a monitor 18 for displaying an endoscopic image outputted from the processor 16; and an input unit 20 for receiving input operations.

The endoscopic diagnosis system 10 is capable of normal light observation mode (white light observation mode) for illuminating the subject with normal light (white light) and imaging the reflected light thereof to display (observe) a normal light image (white light image, reflected light image) and autofluorescence observation mode (special light observation mode) for irradiating the subject with excitation light used for autofluorescence observation (special light) and imaging the autofluorescence to display an autofluorescence image (special light image). The observation mode is switched as appropriate according to an instruction entered from a selector switch 66 of the endoscope device 14 or the input unit 20.

The light source device 12 comprises a light source controller 22, two kinds of laser light sources LD1, LD2 for emitting laser beams having different central wavelengths, a combiner (multiplexer) 24, and a coupler (demultiplexer) 26.

According to this embodiment, the laser light sources LD1, LD2 emit narrowband light beams having given wavelength ranges (e.g., central wavelength +/- 10 nm) with central wavelengths of 405 nm and 445 nm, respectively. The excitation light sources LD1, LD2 emit excitation light for causing autofluorescent substances in body tissues such as porphyrin, NADH (reduced nicotinamide adenine dinucleotide), NADPH (reduced nicotinamide adenine dinucleotide phosphate) and FAD (flavin adenine dinucleotide) to emit autofluorescence. As described later, the laser light source LD2 is also a white light source that emits excitation light for causing a fluorescent body to emit white light or pseudo white light.

The on/off control and light amount control of the laser light sources LD1, LD2 are made independently of each other by the light source controller 22 controlled by a controller of the processor 16 described later, and the emission timing and the emission amount ratio may be freely varied. The laser light sources LD1, LD2 may be constituted using, among others, broad area type InGaN-based laser diodes as well as InGaNas-based laser diodes and GaNas-based laser diodes.

The normal light source for generating normal light is not limited to a light source using a combination of excitation light and a fluorescent body and may be any light source to generate white light, including, for example, a xenon lamp, a halogen lamp, and a white LED (light emitting diode). The excitation light source for generating excitation light for autofluorescence observation is not limited to a laser light source (semiconductor laser) and may be, for example, a white light source or any other light source as appropriate capable of emitting excitation light having an intensity sufficient to cause an autofluorescent substance to emit autofluorescence combined with a band limiting filter.

The wavelength of the excitation light for normal light observation, i.e., central wavelength and wavelength range of narrowband light, is not specifically limited, and any excitation light capable of causing a fluorescent body to generate pseudo white light may be used. The wavelength of the excitation light for autofluorescence observation is also not specifically limited, and any excitation light having a wavelength capable of causing an autofluorescent substance to generate autofluorescence may be used, preferred excitation light including light having a wavelength range of 370 nm to 470 nm, in particular a wavelength range of 400 nm to 450 nm

In this embodiment, the normal light source acts also as one of the excitation light sources but they may be separate sources. In this embodiment, two excitation light, one having a central wavelength of 405 nm and the other having a central wavelength of 445 nm, are used for autofluorescence observation, but the number of excitation light is not limited to two; one or more excitation light may be used respectively for the autofluorescent substances caused to emit light according to the kinds of autofluorescence.

According to this embodiment, the light source device 12 and a fluorescent body constitute a light source unit in the present invention. The light source unit emits white light and one or more first excitation light having different central wavelengths for causing a plurality of autofluorescent substances contained in an observation region of a subject to emit one or more autofluorescence.

The light source controller 22 turns the laser light source LD1 off and turns the laser light source LD2 on in the normal light observation mode. The light source controller 22 turns on both the laser light sources LD1, LD2 in the autofluorescence observation mode.

The laser beams emitted from the laser light sources LD1, LD2 are passed through condenser lenses (not shown) to enter their respective optical fibers, combined by the combiner 24 and divided into four channels of light beams by the coupler 26 before being transmitted to a connector unit 32A. The combiner 24 and the coupler 26 are composed of, for example, a half mirror or a reflection mirror. The configuration is not limited this way; the laser beams from the laser light sources LD1, LD2 may be directly transmitted to the connector unit 32A in lieu of through the combiner 24 and the coupler 26.

The endoscope device 14 is an electronic endoscope instrument comprising an optical system for illumination for emitting four channels (four beams) of light (normal light or excitation light for autofluorescence observation) from the tip of the endoscope insertion section that is inserted into the inside of the subject's body and two channels (two sensors) of optical imaging systems for acquiring an endoscopic image of the observation region. The endoscope device 14 comprises the endoscope insertion section 28, an operating unit 30 for bending the tip of the endoscope insertion section 28 and performing an operation for observation, and connectors 32A, 32B for detachably connecting the endoscope device 14 to the light source device 12 and the processor 16.

The endoscope insertion section 28 comprises a soft portion 34 having a flexibility, a bending portion 36, and a tip (also referred to below as endoscope tip portion) 38.

The bending portion 36 is provided between the flexible portion 34 and the tip 38 and can be bent by rotating an angle knob 40 provided on the operating unit 30. The bending portion 36 can be bent in any direction and to any angle according to, for example, the subject's site for which the endoscope device 14 is used to observe, so that the endoscope tip portion 38 may be directed toward a desired site for observation.

The operating unit 30 and the endoscope insertion section 28 contain therein a forceps channel for inserting, for example, a tissue collecting tool; air supply/water supply channels, and other channels, not shown.

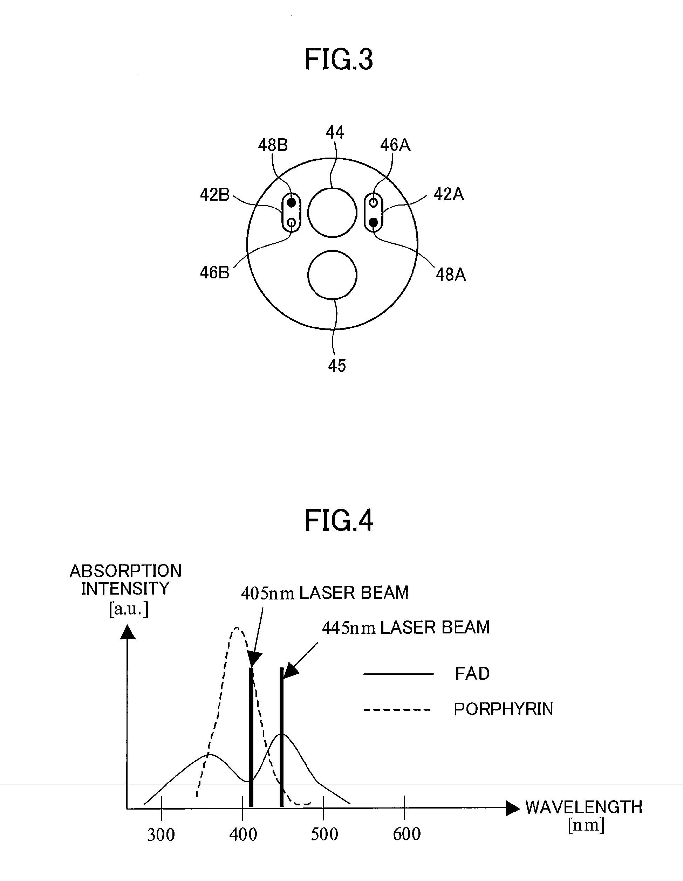

At the tip end surface of the endoscope tip portion 38 are provided two channels of illumination windows 42A, 42B for illuminating an observation region and one channel of observation window 44 for imaging the reflected light or autofluorescence from the observation region as well as a forceps aperture 45 as illustrated in Fig. 3.

On the inside of the illumination window 42A are provided two channels of optical fibers 46A, 48A. The optical fibers 46A, 48A extend from the light source device 12 through the connector unit 32A to the scope tip portion 38. The optical fiber 46A has at its tip portion (its end closer to the illumination window 42A) an optical system including, for example, a lens 50A. The optical fiber 48A has at its tip portion a fluorescent body 54A, and beyond the fluorescent body 54A is provided an optical system including, for example, a lens 52A.

On the inside of the illumination window 42B are likewise provided two channels of optical fibers: an optical fiber 46B having at its tip portion an optical system including, for example, a lens 50B; and an optical fiber 48B having at its tip portion an optical system including, for example, a fluorescent body 54B and a lens 52B.

Fluorescent bodies 54A, 54B comprise a plurality of kinds of fluorescent substances that emit green to yellow light when excited upon absorbing part of the blue laser beam emitted from the laser light source LD2 (e.g., YAG-based fluorescent substance or a fluorescent substance such as BAM ($BaMgAl_{10}O_{17}$)). When the excitation light for normal light observation irradiates the fluorescent bodies 54A, 54B, the green to yellow light generated upon excitation (fluorescence) emitted from the fluorescent bodies 54A, 54B is allowed to blend with part of the blue laser beam that is passed through and not absorbed by the fluorescent bodies 54A, 54B to generate white light (pseudo white light).

The optical system for illumination comprising the illumination window 42A and the optical system for illumination comprising the illumination window 42B have an equivalent configuration and operations; equivalent illumination light simultaneously emitted from the illumination windows 42A, 42B prevent occurrence of uneven illumination. The illumination windows 42A, 42B may emit different illumination light. Provision of optical systems for illumination that emit four channels of illumination light is not essential; an optical system for illumination to emit two or one channel of illumination light, for example, may be used to achieve an equivalent function.

On the inside of the observation window 44 is installed an optical system including, for example, a lens 56; behind the lens 56 is provided a half mirror 57. Further along the optical path of the transmitted light passing through the half mirror 57 and the optical path of the reflected light reflected by the half mirror 57 are provided image sensors 58A, 58B, which may be exemplified by a CCD (charge coupled device) image sensor and a CMOS (complementary metal-oxide semiconductor), for acquiring image information of an observation region. The image sensor 58A, a normal sensor, is used for normal light observation; the image sensor 58B, a high-sensitivity sensor, is used for autofluorescence observation. Since the signal intensity or fluorescence intensity of autofluorescence is weak, the image sensor 58B used in this embodiment for autofluorescence observation has a higher sensitivity than the image sensor 58A used for normal light observation.

Directing the received light to the image sensor 58A or the image sensor 58B need not necessarily be achieved by the half mirror 57; instead, use may be made of, for example, a total reflection mirror that is protruded onto and retracted from the optical path of the received light.

The image sensors 58A, 58B receive light (transmitted light, reflected light) from the lens 56 at their light receiving surface and photoelectrically convert the received light and output an imaging signal, an analog signal. The image sensors 58A, 58B have sets of pixels arranged in matrix, one set of pixels being composed of pixels of three colors, an R pixel, a G pixel, and a B pixel. The image sensor 58A is provided on its light receiving surface (optical path) with R, G, and B color filters corresponding to the R pixels, G pixels, and B pixels, having a spectral transmission characteristic whereby reflected light in a wavelength range of about 370 nm to 720 nm of the visible light delivered from the observation region is divided into three for transmission. The image sensor 58B is provided on its light receiving surface (optical path) with R, G, and B color filters corresponding to the R pixels, G pixels, and B pixels, having a spectral transmission characteristic whereby red autofluorescence and green autofluorescence in a wavelength range of about 500 nm to 700 nm emitted from an autofluorescent substance are transmitted, while the excitation light is blocked.

The image sensors 58A, 58B of this embodiment constitute an imaging unit in the present invention. When the observation region of the subject is illuminated with white light emitted from the light source unit, the imaging unit of the invention receives reflected light of the white light from the observation region of the subject to acquire a reflected light image; when the observation region of the subject is irradiated with excitation light used for autofluorescence observation emitted from the light source unit, the imaging unit receives the autofluorescence emitted from an autofluorescent substance contained in the observation region of the subject to acquire an autofluorescence image.

The color filters constitute a wavelength selection member of the invention. The wavelength selection member is provided on the optical path of the image sensors to transmit light having wavelength ranges corresponding to green and red light. The wavelength selection member is not limited to the above color filters and may also be constituted using, for example, an etalon described in JP 2009-95683 A. Where the etalon is used, the wavelength range of green light may be 500 nm to 600 nm, and the wavelength range of red light may be 610 nm to 650 nm.

This embodiment uses two image sensors, the image sensor 58A for acquiring a normal light image (reflected light image) and the image sensor 58B for acquiring an autofluorescence image, but one may use, for example, one image sensor to acquire both a normal light image and an autofluorescence image.

The light emitted from the light source device 12 and guided through the optical fibers 46A, 46B and 48A, 48B is emitted from the endoscope tip portion 38 toward the subject's observation region. The reflected light from an illuminated observation region or autofluorescence emitted from an autofluorescent substance in an observation region is caused by the lens 56 to form an image on the light receiving surfaces of the image sensors 58A, 58B and undergoes photoelectric conversion through the image sensors 58A, 58B for imaging. The image sensors 58A, 58B output imaging signals (analog signals) of the subject's imaged observation region.

In the normal light observation mode, the excitation light used for the normal light observation emitted from the light source LD2 is guided through the optical fibers 48A, 48B to irradiate the fluorescent bodies 54A, 54B, whereupon the white light generated from the fluorescent bodies 54A, 54B is emitted from the illumination windows 42A, 42B to illuminate the subject's observation region. The reflected light from the subject's observation region illuminated with white light is condensed by the lens 56 and dispersed by the color filters, whereupon the normal light image (reflected light image) is acquired by the image sensor 58A.

In the autofluorescence observation mode, the excitation light used for the autofluorescence observation emitted from both the laser light sources LD1, LD2 are guided through the optical fibers 46A, 46B and emitted from the endoscope tip portion 38 toward the subject's observation region. The autofluorescence emitted from the autofluorescent substance in the subject's observation region irradiated with the excitation light is condensed by the lens 56 and dispersed by the color filters, whereupon the autofluorescence images in green and red are acquired by the image sensor 58B.

Imaging signals (analog signals) of images (normal light image and autofluorescence image) outputted from the image sensors 58A, 58B are delivered through scope cables 62A, 62B to enter A/D converters 64A, 64B. The A/D converters 64A, 64B convert the imaging signals (analog signals) supplied from the image sensors 58A, 58B into image signals (digital signals). The image signals obtained through the conversion pass through the connector unit 32B to enter an image processor 70 of the processor 16.

The processor 16 comprises the controller 68, the image processor 70, and a storage 72. The controller 68 is connected to the monitor 18 and the input unit 20. The processor 16 controls the light source controller 22 of the light source device 12 according to an instruction inputted from the selector switch 66 of the endoscope device 14 and the input unit 20 and image-processes image signals inputted from the endoscope device 14 to produce and output a display image to the monitor 18.

The controller 68 controls the operations of the image processor 70 and the light source controller 22 of the light source device 12 according to instructions, such as an observation mode instruction, given by the selector switch 66 of the endoscope device 14 and the input unit 20.

The image processor 70 performs a given image processing on the image signals entered from the endoscope device 14 under the control of the controller 68 according to the observation mode depending on the kinds of images: a normal light image and an autofluorescence image. The image processor 70 comprises a normal light image processor 70A and an autofluorescence image processor 70B.

The normal light image processor 70A performs given image processing on the image signals (image data) of the normal light image supplied from the A/D converter 64A in the normal light observation mode and outputs (produces) a normal light image signal (normal light image).

The normal light image processor 70B performs given image processing of the image signals (image data) of the autofluorescence image supplied from the A/D converter 64B in the autofluorescence observation mode and outputs (produces) autofluorescence image signals (autofluorescence image). The autofluorescence image processor 70B corrects the signal intensity of the autofluorescence image according to the reflectance of the normal light image (reflected light image) to remove the effects of light absorption by blood from the autofluorescence image.

The image processor 70 in this embodiment constitutes an image correction unit. The image correction unit of the invention has correction coefficients for correcting the signal intensity of the autofluorescence image for decrease occurring in accordance with the amount of blood as the autofluorescence emitted from the autofluorescent substance contained in the subject's observation region is absorbed by blood. The image correction unit obtains a correction coefficient from among correction coefficients corresponding to the reflectance of the reflected light image and uses the obtained correction coefficient to correct the signal intensity of the autofluorescence image.

Further, the image correction unit corrects the non-linearity occurring between the reflectance of the reflected light image and the signal intensity of the autofluorescence image according to the difference between a first propagation length over which the reflected light of the white light, occurring when the white light hits the subject's observation region, travels from the subject's observation region until the reflected light is received by the imaging unit and a second propagation length over which the autofluorescence emitted from the autofluorescent substance contained in the subject's observation region when the first excitation light hits the subject's observation region travels until the autofluorescence is received by the imaging unit.

The image signals processed by the image processor 70 are sent to the controller 68. The controller 68 causes the normal light image or a synthesized image of the normal light image and the autofluorescence image to be displayed on the monitor 18 based on the normal light image signals and the autofluorescence image signals according to the observation mode. The controller 68 assigns the green image signal $G^1$ of a corrected autofluorescence image to the G channel and the red image signal R' to the R channel and the B channel so that the monitor 18 displays the corrected autofluorescence image in pseudo color.

Under the control of the controller 68, the normal light image signals and the autofluorescence image signals are stored in a storage 72 constituted by a memory or other like storage unit, for example, in a unit of one frame of image as required.

Light absorption by blood is now described below.

Fig. 4 is a graph of an example of light absorption intensity characteristics of autofluorescent substances. In the graph, the vertical axis shows light absorption intensity of autofluorescent substances (a.u.: any unit), and the horizontal axis shows wavelength (nm). The graph shows absorption intensity characteristics of FAD and porphyrin, both being autofluorescent substances having a correlation with tumor. The graph also shows central wavelengths 405 nm and 445 nm of laser beams used as excitation light for autofluorescence observation in this embodiment.

FAD has a characteristic of absorbing light having a wavelength range of about 270 nm to 540 nm. The light absorption intensity of FAD gradually increases as the wavelength increases from about 270 nm, and reaches a first peak at a wavelength of about 380 nm, thereafter gradually decreases as the wavelength increases, reaching a minimum at a wavelength of about 420 nm. The absorption intensity then gradually increases again as the wavelength increases from about 420 nm and reaches a second peak at a wavelength of about 460 nm, thereafter gradually decreasing as the wavelength increases.

Porphyrin has a characteristic of absorbing light having a wavelength range of about 340 nm to 450 nm. Porphyrin has a light absorption intensity characteristic of peaking at a wavelength of about 390 nm and gradually decreasing as the wavelength decreases or increases.

As will be seen from this graph, irradiation of the subject with a laser beam having a central wavelength of 405 nm excites mostly porphyrin in the observation region to generate autofluorescence. Further, irradiation of the subject with a laser beam having a central wavelength of 445 nm excites mostly FAD in the observation region to generate autofluorescence.

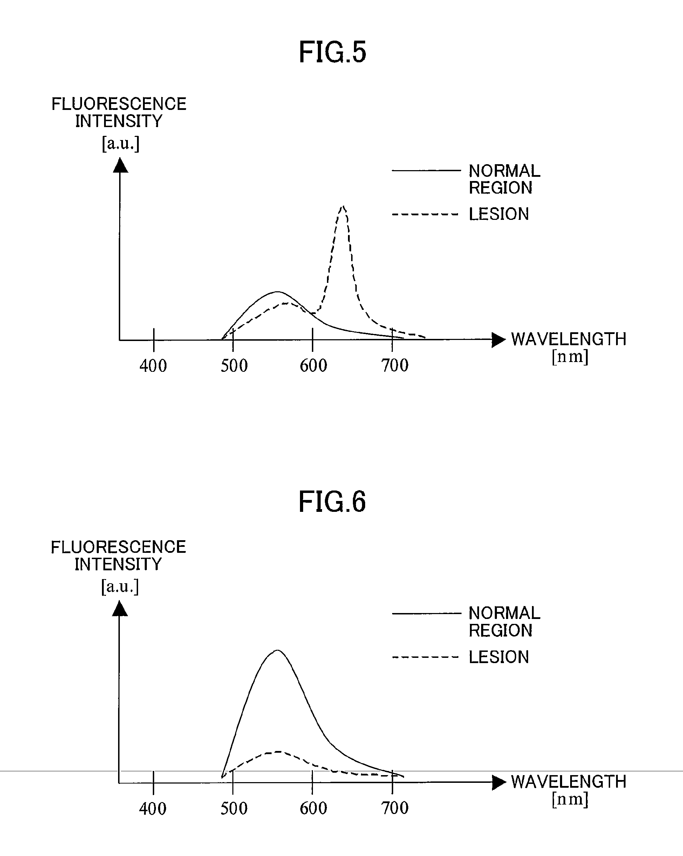

Fig. 5 is a graph of an example of fluorescence intensity characteristics of autofluorescent substances. In the graph, the vertical axis shows fluorescence intensity of autofluorescent substances (a.u.), and the horizontal axis shows wavelength (nm). The graph, corresponding to the graph of Fig. 4, shows a fluorescence intensity distribution of autofluorescence emitted from an autofluorescent substance in a normal region and a lesion when the subject's observation region is irradiated with laser beams having a central wavelength of 405 nm used as excitation light for the autofluorescence observation.

Irradiation of the subject with a laser beam having a central wavelength of 405 nm as excitation light for autofluorescence observation mostly excites porphyrin as earlier described, and, as illustrated in Fig. 5, the observation region irradiated with the excitation light emits autofluorescence having a wavelength range of about 480 nm to 740 nm.

The fluorescence intensity of the lesion gradually increases as the wavelength increases from about 480 nm, reaches a first peak at a wavelength of about 560 nm and thereafter gradually decreases as the wavelength increases, reaching a minimum at a wavelength of about 610 nm. The fluorescence intensity then gradually increases again as the wavelength increases from about 610 nm and reaches a second peak at a wavelength of about 630 nm, thereafter gradually decreasing as the wavelength increases. A range close to the second peak represents autofluorescence emitted mostly from porphyrin.

On the other hand, the fluorescence intensity of the normal region gradually increases as the wavelength increases from about 480 nm and reaches a peak at a wavelength of about 550 nm, thereafter gradually decreasing as the wavelength increases. A region close to the peak represents autofluorescence emitted mostly from FAD.

It is known that porphyrin accumulates in a lesion. As Fig. 5 shows, the fluorescence intensity of porphyrin is higher in the lesion than in the normal region. Therefore, knowing how the fluorescence intensity varies enables distinction between normal region and lesion (reference : "Hikari niyoru Igaku Shindan," 6th Vol., in a series "Hikariga Hiraku Seimei Kagaku," by Mamoru TAMURA, edited by Photobiology Association of Japan, published by KYORITSU SHUPPAN CO., LTD., March 18, 2001).

The graph illustrated in Fig. 6 shows a fluorescence intensity distribution of autofluorescence emitted from an autofluorescent substance in a normal region and a lesion when the subject's observation region is irradiated with laser beams having a central wavelength of 445 nm used as excitation light for the autofluorescence observation.

Irradiation of the subject with a laser beam having a central wavelength of 445 nm as excitation light for autofluorescence observation mostly excites FAD as earlier described, and, as illustrated in Fig. 6, the observation region irradiated with the excitation light emits autofluorescence having a wavelength range of about 480 nm to 720 nm.

The fluorescence intensity gradually increase in both the lesion and the normal region as the wavelength increases from about 480 nm and reaches a peak at a wavelength of about 550 nm, thereafter gradually decreasing as the wavelength increases. A region close to the peak represents autofluorescence emitted mostly from FAD.

As illustrated in Fig. 6, the fluorescence intensity of FAD is lower in the lesion than in the normal region. Therefore, knowing how the fluorescence intensity of FAD varies likewise enables distinction between normal region and lesion.

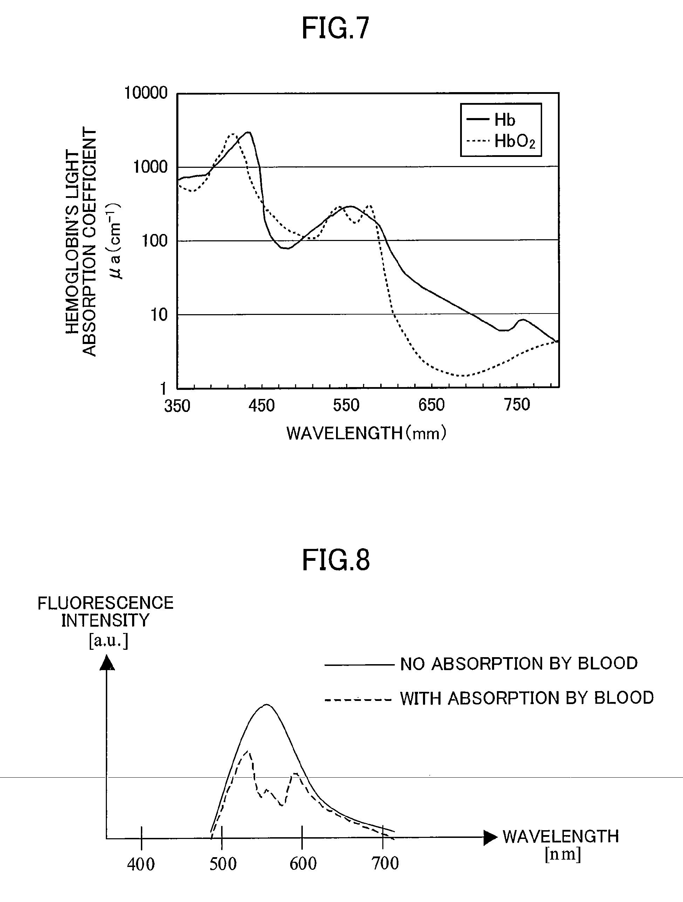

Fig. 7 is a graph showing light absorption coefficients of oxygenated hemoglobin and reduced hemoglobin. In the graph, the vertical axis shows hemoglobin's light absorption coefficient $\mu a(cm^{-1})$, the horizontal axis wavelength (nm). As illustrated in the graph, the blood hemoglobin has light absorption characteristics such that the light absorption coefficient $\mu a$ changes according to the wavelength of light used for illumination. The light absorption coefficient $\mu a$ denotes an absorbance representing the magnitude of light absorption by hemoglobin. A reduced hemoglobin Hb not combined with oxygen and an oxygenated hemoglobin $HbO_2$ have different light absorption characteristics such that they have different absorbances except at isosbestic points at which both exhibit the same light absorbance (absorption coefficient $\mu a$) (intersections of light absorption characteristics curves of reduced hemoglobin and oxygenated hemoglobin in Fig. 7). Generally, the distributions illustrated in Fig. 7 change into a non-linear line depending on the region of a subject to be imaged and, therefore, need to have been previously obtained by, for example, measuring an actual body tissue or conducting a simulation of light propagation.

Fig. 8 is a graph illustrating an example of fluorescence intensity characteristics of FAD each in the presence and absence of blood. The graph shows autofluorescence intensity distributions of autofluorescence emitted from the autofluorescent substances in the normal region shown in Fig. 6, that is, emitted mostly from FAD. Fig. 7 shows that the light absorption by blood peaks near a wavelength of 550 nm. The wavelength at which FAD-emitted autofluorescence peaks at a wavelength of about 550 nm as is apparent from Figs. 5 and 6 and coincides with that at which the hemoglobin light absorption peaks. Accordingly, as illustrated by the graph of Fig. 8, where, for example, blood vessels lie above FAD in a body tissue, the FAD autofluorescence spectrum exhibits decrease in autofluorescence intensity according to the light absorption by blood.

To eliminate the effects of light absorption by blood, the amount of light absorption by blood may be obtained from the reflectance (signal intensity) of a reflected light image in the same wavelength range as the emission wavelength of the autofluorescence. However, the relation between fluorescence intensity and reflectance is not a simple linear relation because of the difference in propagation length in body tissue between autofluorescence and reflected light. Reflected light is the very light observed as it , after being admitted into a body tissue and scattering therein, travels back from the body tissue into the outside thereof. The autofluorescence being observed is autofluorescence as it travels to the outside of the body tissue after light is admitted into the body tissue, scatters therein, and hits the autofluorescent substance, only then causing the autofluorescence to be generated, the autofluorescence then scattering in the body tissue before leaving the body tissue.

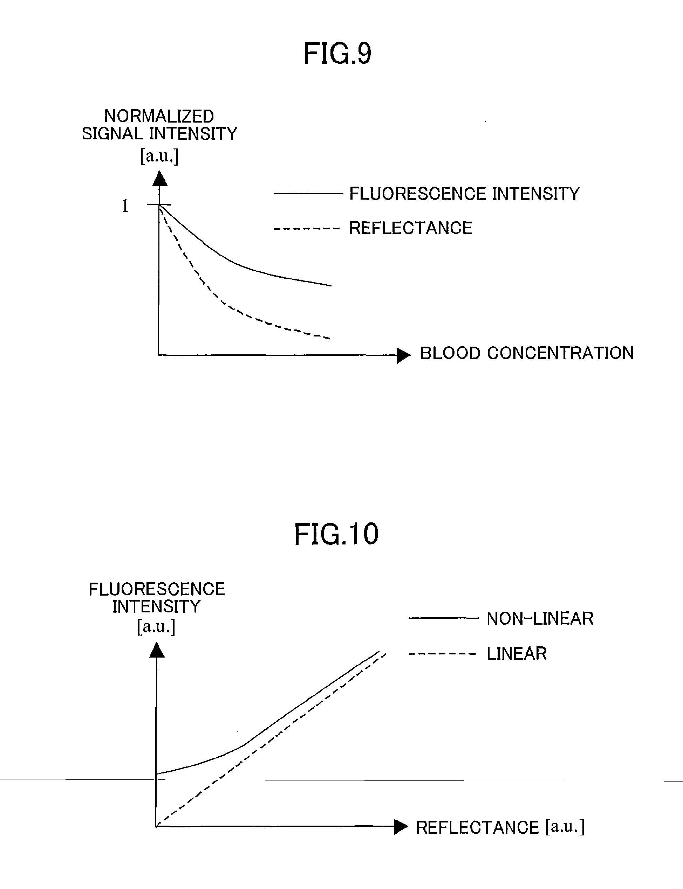

Fig. 9 is a graph illustrating an example of relation between reflectance and fluorescence intensity and blood concentration. In the graph, the vertical axis shows a normalized signal intensity (a.u.) of reflectance (reflected light) and fluorescence intensity (autofluorescence); the horizontal axis shows blood concentration. The graph shown in that figure is normalized such that when the blood concentration is 0, both autofluorescence and reflected light are 1. Fig. 9 shows that when light, scattering in a body tissue, chances on a region containing blood such as, for example, a blood vessel, the signal intensity of the light is known to weaken due to light absorption by blood. In particular, as the blood concentration increases, the signal intensity of reflected light (reflectance) weakens more significantly than that (fluorescence intensity) of the autofluorescence. Reflected light, which is generally scattered over a longer distance in the body tissue as described above, tends to be affected more by light absorption by blood than autofluorescence.

Fig. 10 is a graph illustrating an example of relation between fluorescence intensity and reflectance. In the graph, the vertical axis shows fluorescence intensity (a.u.); the horizontal axis shows reflectance (a.u.). As shown in the dotted line in the graph, if the autofluorescence and the reflected light are scattered over the same distance in a body tissue, the autofluorescence and the reflected light are in a linear relation. As described above, however, because reflected light is generally scattered over a longer distance in a body tissue than autofluorescence, both are in a non-linear relation as shown in the solid line in the graph. The non-linearity increases in particular in a region with a high blood concentration and a low reflectance.

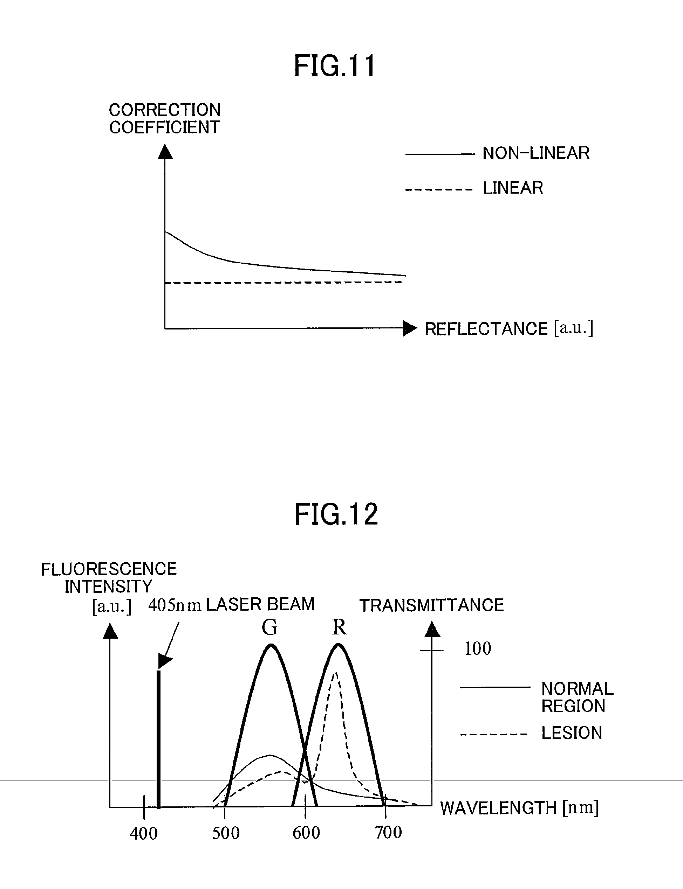

Fig. 11 is a graph illustrating an example of correction coefficients for correcting the fluorescence intensity of autofluorescence. In the graph, the vertical axis shows correction coefficient; the horizontal axis shows reflectance (a.u.). As shown in the dotted line in the graph, if the autofluorescence and the reflected light are in a linear relation, the correction coefficient has a constant value regardless of the reflectance. However, both are in a non-linear relation as shown in the solid line in the graph, and because the non-linearity increases in particular in a region with a low reflectance, the correction coefficient is so set as to be higher for a region with a low reflectance than for a region with a high reflectance. Thus, a correction coefficient corresponding to a reflectance is used to correct the fluorescence intensity corresponding to that reflectance, making it possible to correct the non-linearity between them and eliminate the effects of light absorption by blood from the autofluorescence image.

The correction coefficient shown in Fig. 11 can be previously calculated experimentally. For example, a scattering substance (a solution having the same scattering characteristic as the organism, such as a 1% intralipid solution) and an autofluorescent substance (FAD or porphyrin having a concentration of about 10 $\mu$mol, which is close to the concentrations of those substances in the body tissue) are placed in a container such as a Petri dish. Then, the reflected light and the autofluorescence are imaged as the blood concentration (hemoglobin concentration) is allowed to change in a range of 0 mg/dl to 300 mg/dl. Then, a graph shown in Fig. 10 is produced for image signals of the G channel having the same wavelength range as the FAD's emission wavelength and of the R channel having the same wavelength range as the porphyrin's emission wavelength, and the correction coefficient illustrated in Fig. 11 can be produced for the image signals of the G and the R channel from the difference from the linear line shown in Fig. 10 so that the fluorescence intensity remains a constant value.

A correction coefficient table storing relations between reflectance of the reflected light image and correction coefficient may be used to obtain the correction coefficient corresponding to the reflectance of the reflected light image, or a calculation function for obtaining the correction coefficient may be used in lieu of the correction coefficient table.

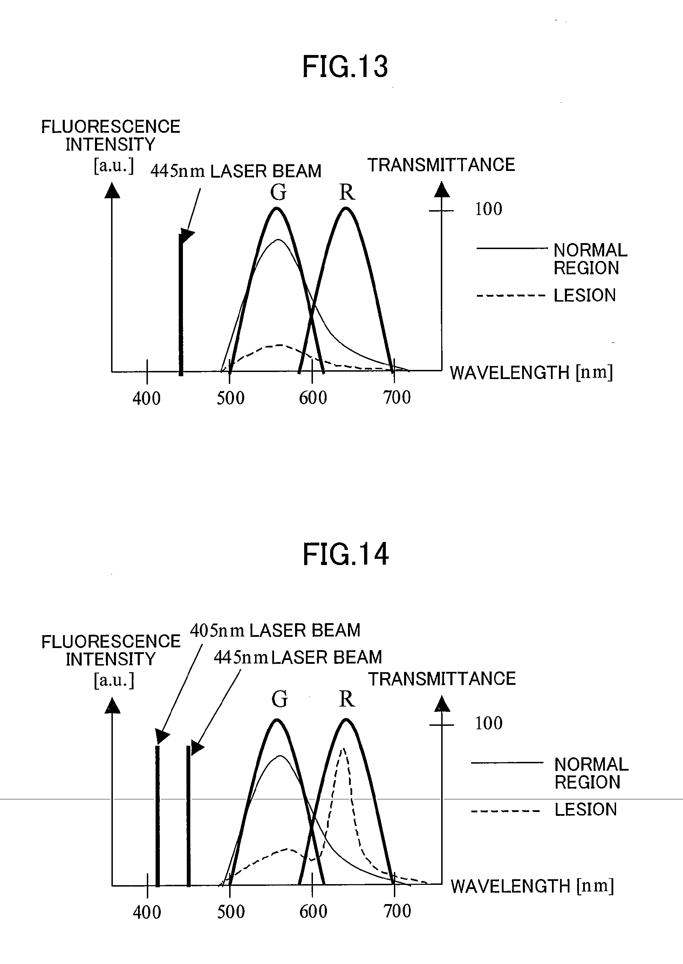

Figs. 12 and 13 are graphs where spectral transmission characteristics of color filters provided at the light receiving surface of the image sensor 58B are superimposed over the graphs of Figs. 5 and 6, respectively. The spectral transmission rates (%) of the color filters are shown on the vertical axis on the right side in the graphs. The graphs also show central wavelengths 405 nm and 445 nm of laser beams used as excitation light for the autofluorescence observation.

As shown in these graphs, the green color filters are designed to transmit light having a wavelength range of 500 nm to 620 nm with a central wavelength at about 550 nm. Thus, the autofluorescence emitted mainly from FAD passes through the green color filters and undergoes photoelectric conversion through the image sensor 58B. The red color filters are designed to transmit light having a wavelength range of 580 nm to 700 nm with a central wavelength at about 630 nm. Thus, the autofluorescence emitted mainly from porphyrin passes through the red color filters and undergoes photoelectric conversion through the image sensor 58B.

The invention is not limited to the above, however; the green color filters preferably transmit light having a wavelength range of 500 nm to 600 nm; the red color filters preferably transmit light having a wavelength range of 600 nm to 700 nm.

As described above, the image sensor 58B is not provided with blue color filters. Thus, the laser beams serving as excitation light for autofluorescence observation having central wavelengths of 405 nm and 445 nm are cut off and do not undergo photoelectric conversion through the image sensor 58B.

Fig. 14 is a graph of autofluorescence's fluorescence intensity distributions obtained when the laser beams serving as excitation light for autofluorescence observation having central wavelengths of 405 nm and 445 nm are emitted simultaneously, with spectral transmission characteristics of the color filters superimposed. The graph also shows central wavelengths 405 nm and 445 nm of laser beams used as excitation light for the autofluorescence observation.

It is apparent from the graph that when the laser beams serving as excitation light for autofluorescence observation having central wavelengths of 405 nm and 445 nm are emitted simultaneously, there is produced a significant difference between lesion and normal region in fluorescence intensity of autofluorescence generated mainly from FAD at a wavelength near 550 nm and fluorescence intensity of autofluorescence generated mainly from porphyrin at a wavelength near 630 nm.

Therefore, the autofluorescence is subjected to dispersion through the green and the red filters to obtain a green autofluorescence image and a red autofluorescence image such that the difference in fluorescence intensity of FAD between legion and normal region can be observed in the green autofluorescence image. Likewise, the difference in fluorescence intensity of porphyrin between legion and normal region can be observed in the red autofluorescence image.

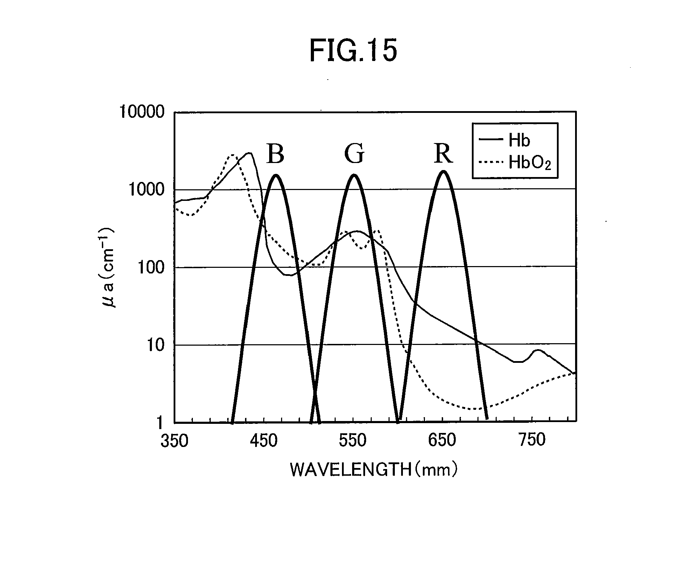

Fig. 15 is a graph illustrating an example of relation between blood's absorption coefficients and color filters in a normal sensor. In the graph, the vertical axis shows hemoglobin's light absorption coefficient $\mu a$ ($cm^{-1}$); the horizontal axis shows wavelength (nm). It is apparent from the graph that the wavelength characteristics of the green and the red color filters provided in a normal sensor are the same as shown in Fig. 14. The green and the red image signal (image data) from a normal sensor yield information on light absorption by blood corresponding to the wavelengths of green and red light.

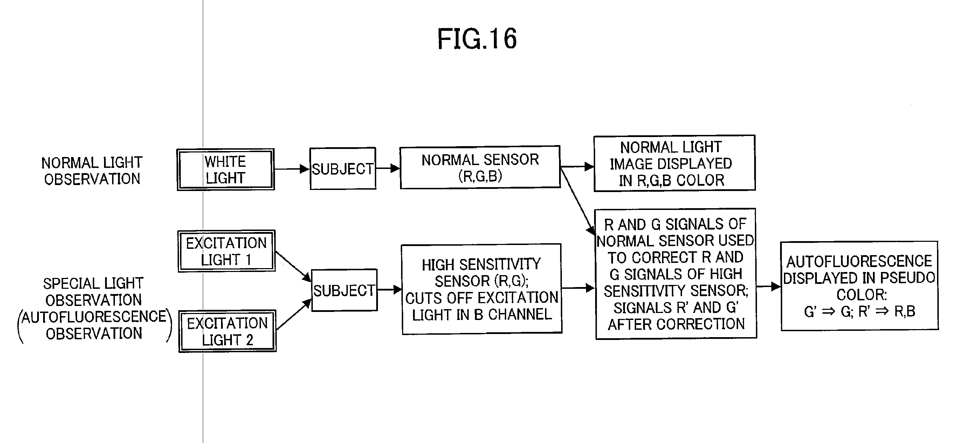

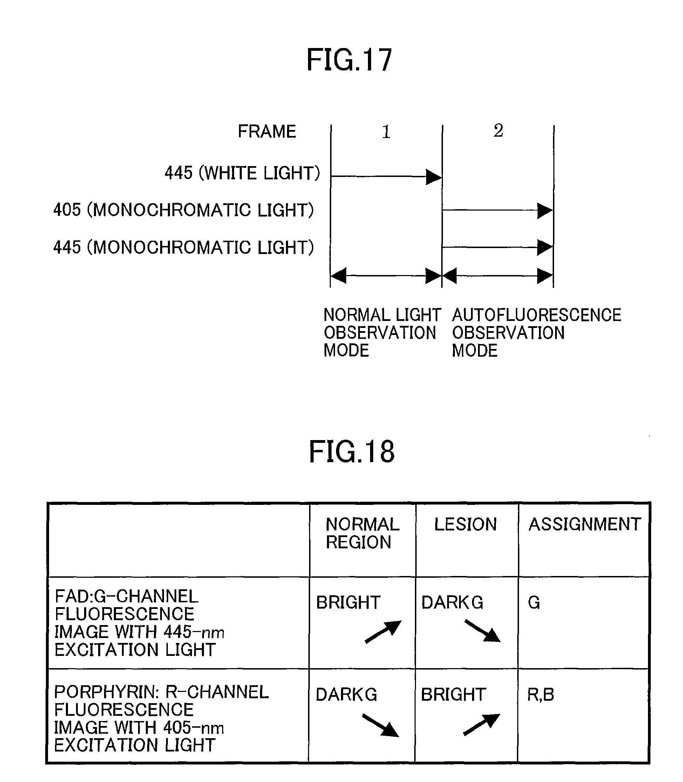

The operation of the endoscopic diagnosis system 10 will now be described with reference to the conceptual views shown in Figs. 16 and 17.

In the normal light observation mode, the light source controller 22 controls the laser light source LD1 to be turned off and the laser light source LD2 to be turned on. The laser beam having a central wavelength of 445 nm emitted from the laser light source LD2 is directed to irradiate the fluorescent bodies 54A, 54B, causing the fluorescent bodies 54A, 54B to emit white light. As shown in Fig. 16, the white light emitted from the fluorescent bodies 54A, 54B are directed to illuminate the subject, the reflected light is received by the image sensor 58A (normal sensor), and a normal light image containing R, G, and B channel image signals is acquired. The normal light image is displayed in color based on the R, G, and B channel image signals (normal light image processing).

In the autofluorescence observation mode, acquisition of images is performed repeatedly in units of, for example, two frames as illustrated in Fig. 17. The mode in the first of the two frames is the same observation mode as the normal light observation mode; the mode in the second frame is an observation mode unique to the autofluorescence observation mode.

First, in the normal light observation mode in the first frame, a normal light image containing R, G, and B channel image signals is acquired as described above. The normal light image signals are stored in the storage 72 under the control of the controller 68.

In the autofluorescence observation mode in the second frame to follow, both laser light sources LD1, LD2 are turned on under the control of the controller 22 as illustrated in Fig. 17. As illustrated in Fig. 16, when the laser beam having a central wavelength of 405 nm (excitation light 1) emitted from the laser light source LD1 and the laser beam having a central wavelength of 445 nm (excitation light 2) emitted from the laser light source LD2 are allowed to simultaneously irradiate the subject, autofluorescence emitted from the subject is received by the image sensor 58B (high-sensitivity sensor), and an autofluorescence image containing R and G channel image signals are acquired. The autofluorescence image signals are stored in the storage 72 under the control of the controller 68.

As described above, the image sensor 58B is not provided with blue color filters but only provided with green and red color filters. Therefore, the excitation light having central wavelengths of 405 nm and 445 nm, a wavelength range of blue light, is cut off, so that autofluorescence having a wavelength range of 500 nm to 700 nm, a wavelength range of green and red light, can be received by the image sensor 58B.

Subsequently, the autofluorescence image processor 70B uses the normal light image signals and the autofluorescence image signals stored in the storage 72 to obtain correction coefficients respectively corresponding to R and G channel image signals (reflectances) of the normal light image (reflected light image) from a correction coefficient table having, for example, the relation shown by the graph of Fig. 11. Then, the obtained correction coefficients are used to correct the signal intensities of the R and the G channel of the autofluorescence image. Thus, the effects of light absorption by blood in the R and the G channel of the autofluorescence image are eliminated. Now, let R' and G' be the R and the G channel image signal of the corrected autofluorescence image, respectively.

The normal light image corresponding to the normal light image signals stored in the storage 72 and the corrected autofluorescence image are combined, and their combined image is displayed on the monitor 18. The controller 68 assigns the green image signal G' of a corrected autofluorescence image to the G channel, and the red image signal R' to the R channel and the B channel, so that the autofluorescence image is displayed on the monitor 18 in pseudo color (autofluorescence image processing).

As illustrated in Fig. 18, as the fluorescence intensity of FAD increases, that of the porphyrin decreases in a normal region, and the relation reverses in a lesion. As described above, in the endoscopic diagnosis system 10, the G channel image signal is assigned to the G channel, and the R channel image signal is assigned to the R channel and the B channel to achieve pseudo color display. Accordingly, the pseudo color representation of the autofluorescence image by the endoscopic diagnosis system 10 shows the normal region in green and the lesion in magenta such that the lesion can be represented in higher contrast than with a single autofluorescence image obtained by imaging the autofluorescence emitted from FAD and porphyrin, thus enabling easier recognition of the lesion.

As described above, with the endoscopic diagnosis system 10, an autofluorescence image free from effects of light absorption by blood may be obtained by correcting the intensity of the autofluorescence image signals using a correction coefficient corresponding to the reflectance of a reflected light image having the same wavelength region as the emission wavelength region of the autofluorescence.

In the autofluorescence observation mode, repeated image acquisition in units of two frames is not essential. Nor is simultaneous irradiation with the laser beam having a central wavelength of 405 nm and the laser beam having a central wavelength of 445 nm in the autofluorescence observation mode essential; for example, these laser beams may sequentially irradiate the subject in each frame. In pseudo color display of the autofluorescence image, which color channel of image signal is assigned to which color channel may be arbitrarily determined. The autofluorescent substances are not limited to porphyrin and FAD.

Next, the endoscopic diagnosis system according to a second aspect of the invention is described.

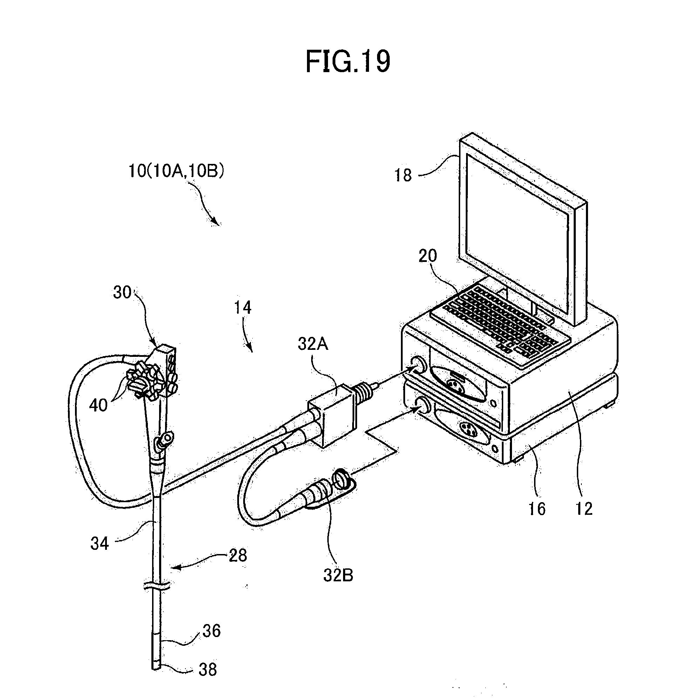

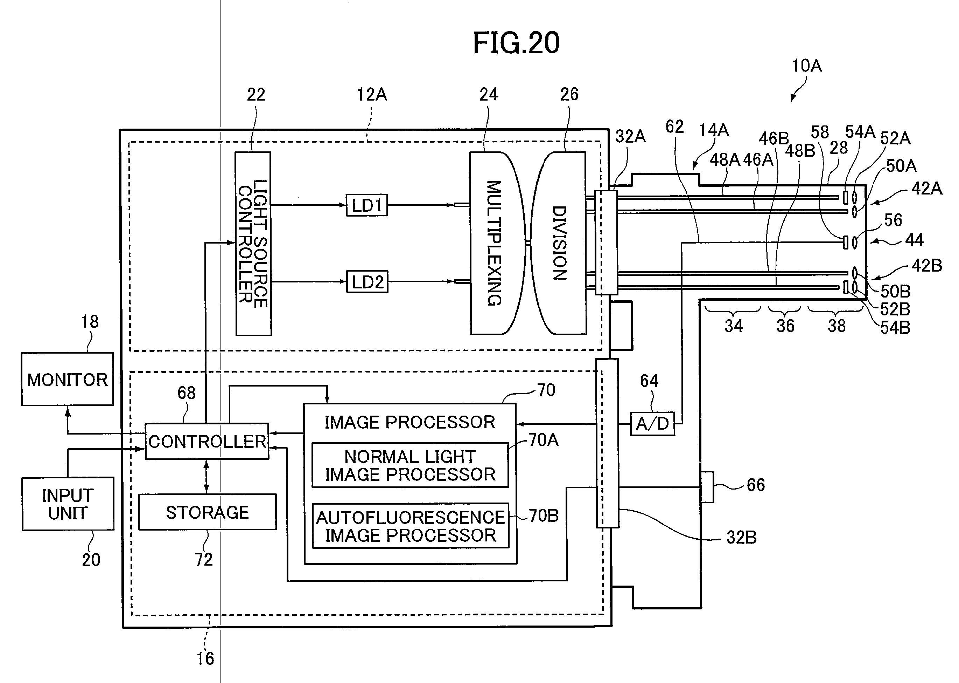

Fig. 19 is an external view of an embodiment illustrating a configuration of an endoscopic diagnosis system according to the second aspect of the invention; Fig. 20 is a block diagram of a first embodiment illustrating an internal configuration of the system shown in Fig. 19. An endoscopic diagnosis system 10A illustrated in that drawing has the same configuration as the endoscopic diagnosis system 10 according to the first aspect shown in Figs. 1 and 2 except that the light source device 12 and the endoscope device 14 are respectively replaced with a light source device 12A and an endoscope device 14A. In the description to follow, like components are given like reference characteristics and are not described in detail in the following description, and focus is placed on different components.

Likewise, the endoscopic diagnosis system 10A is capable of normal light observation mode and autofluorescence observation mode.

According to this embodiment, the laser light sources LD1, LD2 emit narrowband light beams having given wavelength ranges (e.g., central wavelength +/- 10 nm) with central wavelengths of 405 nm and 445 nm, respectively. The laser light source LD1 emits excitation light for causing an autofluorescent substance in a body tissue to emit autofluorescence. The laser light source LD2 is a white light source that emits excitation light for causing a fluorescent body to emit white light (pseudo white light).

This embodiment uses excitation light having a central wavelength of 405 nm for autofluorescence observation, but the number of excitation light is not limited to one; one or more excitation light may be used respectively to emit light according to the kinds of autofluorescence.

In this embodiment, the light source device 12A and a fluorescent body constitute part of a light source unit in the present invention. The light source unit of the invention emits illumination light not limited to white light and including at least green and red light or blue and green light and one or more kinds of excitation light having different central wavelengths for causing an autofluorescent substance contained in the subject's observation region to emit at least one autofluorescence.

The light source controller 22 turns the laser light source LD1 off and turns the laser light source LD2 on in the normal light observation mode. The light source controller 22 sequentially turns on the laser light sources LD1, LD2 in the autofluorescence observation mode.

The endoscope device 14A is an electronic endoscope comprising an optical system for emitting four channels (four beams) of illumination light (normal light or excitation light for autofluorescence observation) and one channel (one sensor) of optical imaging system.

On the inside of the observation window 44 is installed an optical system including, for example, a lens 56; behind the lens 56 is provided an image sensor 58.

The image sensors 58 receives light from the lens 56 at their light receiving surface and photoelectrically converts the received light and outputs an imaging signal, an analog signal. The image sensors 58 has sets of pixels arranged in matrix, one set of pixels being composed of pixels of three colors, an R pixel, a G pixel, and a B pixel. The image sensor 58 is provided on its light receiving surface (on the optical path) with red, green, and blue color filters corresponding to the R pixels, G pixels, and B pixels, having a spectral transmission characteristic of blocking the excitation light having a central wavelength of 405 nm and dividing the reflected light in a wavelength range of about 410 nm to 720 nm of the visible light arriving from the observation region into three for transmission. Thus, the color filters also serve as excitation light cut filters for cutting off light having a central wavelength of, for example, 410 nm or less in order to block excitation light having a central wavelength of 405 nm in the autofluorescence observation mode.

In this embodiment, a single image sensor, the image sensor 58, acquires both the normal light image and the autofluorescence image. According to the invention, however, two or more image sensors may be used: for example, a normal sensor may be used to acquire the normal light image, and a high-sensitivity sensor having a higher sensitivity than the normal sensor may be used to acquire the autofluorescence image.

The light emitted from the light source device 12A and guided through the optical fibers 46A, 46B and 48A, 48B is emitted from the endoscope tip portion 38 toward the subject's observation region. The reflected light from an illuminated observation region or autofluorescence emitted from an autofluorescent substance in an observation region is caused by the lens 56 to form an image on the light receiving surface of the image sensor 58 and undergoes photoelectric conversion through the image sensor 58 for imaging. The image sensor 58 outputs imaging signals (analog signals) of the subject's imaged observation region.

In the normal light observation mode, the excitation light for the normal light observation emitted from the light source LD2 is guided through the optical fibers 48A, 48B to irradiate the fluorescent bodies 54A, 54B, whereupon the white light generated from the fluorescent bodies 54A, 54B is emitted from the illumination windows 42A, 42B to illuminate the subject's observation region. The reflected light from the subject's observation region illuminated with the white light is condensed by the lens 56, and dispersed by the color filters, whereupon the normal light image is acquired by the image sensor 58.

In the autofluorescence observation mode, the excitation light for the autofluorescence observation emitted from the laser light source LD1 is guided through the optical fibers 46A, 46B and emitted from the endoscope tip portion 38 toward the subject's observation region. The autofluorescence emitted from the autofluorescent substance in the subject's observation region irradiated with the excitation light is condensed by the lens 56 and dispersed by the color filters while the excitation is cut off by the color filters, so that the autofluorescence images are acquired by the image sensor 58.

The operation whereby the excitation light for normal light observation is emitted from the laser light source LD2 in the autofluorescence observation mode is the same as in the normal light observation mode.

In the description to follow, R1, G1, and B1 respectively denote the red, the green, and the blue image signal of the autofluorescence image and R2, G2, and B2 denote the red, the green, and the blue image signal of the normal light image.

The imaging signals (analog signals) of images (normal light image and autofluorescence image) outputted from the image sensor 58 are passed through a scope cable 62 to enter an A/D converter 64. The A/D converter 64 converts the imaging signals (analog signals) supplied from the image sensor 58 into image signals (digital signals). The image signals obtained through the conversion pass through the connector unit 32B to enter an image processor 70 of the processor 16.

The processor 16 comprises the controller 68, the image processor 70, and the storage 72. The image processor 70 comprises a normal light image processor 70A and an autofluorescence image processor 70B.

The normal light image processor 70A performs given image processing appropriate for the normal light image on the image signals (image data) of the normal light image supplied from the A/D converter 64 in the normal light observation mode and outputs (produces) normal light image signals (normal light image).

The normal light image processor 70B performs given image processing appropriate for the normal light image on the image signals (image data) of the autofluorescence image supplied from the A/D converter 64 in the autofluorescence observation mode and outputs (produces) autofluorescence image signals (autofluorescence image). The autofluorescence image processor 70B corrects the image signals of the autofluorescence image based on the image signals of the normal light image to remove the effects of light absorption by blood from the autofluorescence image.

The image processor 70 in this embodiment constitutes part of an image correction unit. The image correction unit of the invention contains information on an attenuation ratio with which the excitation light and autofluorescence emitted from an autofluorescent substance contained in a subject's observation region decrease by absorption by blood according to the amount of blood. From the information on the attenuation ratio corresponding to the amount of blood, the image correction unit obtains an attenuation ratio corresponding to the image signals of the reflected light image and uses the obtained attenuation ratio to correct the image signals of the autofluorescence image.

The image signals processed by the image processor 70 are sent to the controller 68. The controller 68 causes the normal light image or a synthesized image of the normal light image and the autofluorescence image to be displayed on the monitor 18 based on the normal light image signals and the autofluorescence image signals according to the observation mode. The controller 68 assigns the green image signal of the corrected autofluorescence image to the G channel and the red image signal to the R channel and the B channel so that the monitor 18 displays the corrected autofluorescence image in pseudo color.

The description to follow explains a method of eliminating the effects of light absorption by blood from the autofluorescence image.

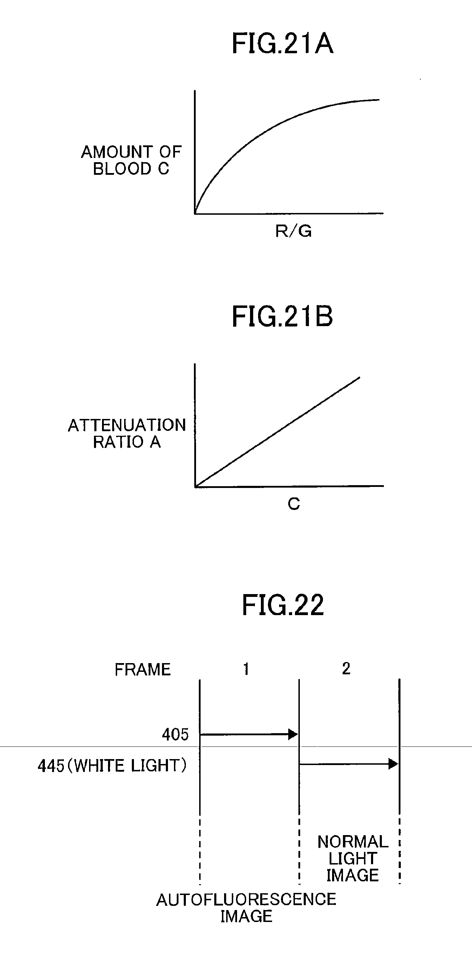

Fig. 21A is a graph of a first conversion table representing a relation between ratio R2/G2 of the red to the green image signal of the normal light image and amount of blood. In the graph, the vertical axis shows amount of blood C; the horizontal axis shows image signal ratio R2/G2. The image signal ratio R2/G2 has a positive correlation with the amount of blood C because of the characteristic of the hemoglobin's light absorption coefficient. It is apparent from the graph that as the image signal ratio R2/G2 increases, the amount of blood C contained in the subject's observation region increases.

Fig. 21B is a graph of a second conversion table representing a relation between amount of blood C and attenuation ratio A. In the graph, the vertical axis shows attenuation ratio A; the horizontal axis shows amount of blood C. As described above, the amount of absorbed excitation light and absorbed autofluorescence or the attenuation ratio A has a positive correlation with the amount of blood C. It is apparent from the graph that as the amount of blood C contained in the subject's observation region increases, the attenuation ratio A increases.

The graphs shown in Figs. 21A and 21B (information on attenuation ratio) can be previously calculated experimentally. These two graphs may be combined into a single graph. The relations represented by these graphs may be represented in the form of, for example, tables and calculation functions.

The amount of blood C is obtained from the graph of Fig. 21A (information on the attenuation ratio corresponding to the image signal ratio R2/G2), and the attenuation ratio A corresponding to the obtained amount of blood C is obtained from the graph of Fig. 21B. The obtained attenuation ratio A is then used to correct the red image signal R1 of the autofluorescence image according to the following formula (1) and obtain the red image signal R1' of the corrected autofluorescence image.

$$R1' = R1/(1 - A) \qquad (1)$$

Thus, the red image signal R1 of the autofluorescence image or, in this embodiment, the component of the autofluorescence emitted mainly from porphyrin, is corrected so that an autofluorescence image corresponding to the magnitude of the amount of the autofluorescent substance can be obtained without regard to the amount of blood C.

While, in the above case, the image signals of the autofluorescence image are corrected based on the image signal ratio R2/G2, use of the image signal ratio R2/G2 is not essential. For example, the image signals of the normal light image (including image signal ratio) related to the amount of blood may be used to correct the image signals of the autofluorescence image. Thus, the image signal ratio G2/B2 or R2/B2, for example, may be used in place of the image signal ratio R2/G2.

While, in the above case, correction is made only of the red image signal R1 of the red autofluorescence image corresponding to the peak emission wavelength, 630 nm, of the autofluorescence emitted from porphyrin, correction of the green image signal G1 may also be made to improve the correction accuracy because, as illustrated in Fig. 5, the autofluorescence emitted from porphyrin also contains the green component. Because the autofluorescence emitted from porphyrin contains hardly any blue component, no problem arises when the blue image signal B1 is not corrected.

In the above case, the red image signal R1 of the autofluorescence image is corrected, but in the case of, for example, FAD instead of porphyrin, it is preferable to preferentially correct the green image signal G1 rather than the red image signal R1 because the central wavelength of the autofluorescence emitted from FAD is 550 nm as illustrated in Fig. 5. Which color of component is to be corrected needs to be thus determined as appropriate according to the autofluorescent substance.

Next, the operation of the endoscopic diagnosis system 10A will be described.

In the normal light observation mode, the light source controller 22 controls the laser light source LD1 to be turned off and the laser light source LD2 to be turned on. The laser beam having a central wavelength of 445 nm emitted from the laser light source LD2 is directed to irradiate the fluorescent bodies 54A, 54B, causing the fluorescent bodies 54A, 54B to emit white light. The white light emitted from the fluorescent bodies 54A, 54B is directed to illuminate the subject, the reflected light is received by the image sensor 58, and the normal light image is thereby acquired. The normal light image is displayed in color based on the blue, green, and red image signals (normal light image processing).

In the autofluorescence observation mode, acquisition of images is performed repeatedly in units of, for example, two frames as illustrated in Fig. 22. In the first of the two frames, the autofluorescence image is acquired; in the second frame, the normal light image is acquired.

In the first frame, the light source controller 22 controls the laser light source LD1 to be turned on and the laser light source LD2 to be turned off. The laser beam having a central wavelength of 405 nm emitted from the laser light source LD1 is directed to irradiate the subject, whereupon the autofluorescence emitted from the subject is received by the image sensor 58 while the excitation light is cut off, acquiring the autofluorescence image. The autofluorescence image signals are stored in the storage 72 under the control of the controller 68.

In the second frame to follow, the light source controller 22 controls the laser light source LD2 to be turned on and the laser light source LD1 to be turned off in order to acquire the normal light image as in the normal light observation mode. The normal light image is stored in the storage 72 under the control of the controller 68.

Subsequently, the autofluorescence image processor 70B uses the autofluorescence image signals and the image signal of the normal light image stored in the storage 72 to obtain the amount of blood C corresponding to the image signal ratio R2/G2 from the graph shown in Fig. 21A and obtain the attenuation ratio A corresponding to the obtained amount of blood C from the graph shown in Fig. 21B. Then, correction is made of the red image signal R1 of the autofluorescence image from the obtained attenuation ratio A using the above formula (1). Thus, the effects of light absorption by blood can be eliminated from the red autofluorescence image signal R1.

The normal light image and the corrected autofluorescence image are combined, and the combined image is displayed on the monitor 18. The controller 68 assigns the green image signal of the corrected autofluorescence image to the G channel, and the red image signal to the R channel and the B channel so that the autofluorescence image is displayed on the monitor 18 in pseudo color (autofluorescence image processing).

As described above, the endoscopic diagnosis system 10A can eliminate the effects of light absorption by blood from the autofluorescence image and distinguish the magnitude of the amount of the autofluorescent substance from the magnitude of light absorption by blood, achieving an enhanced diagnosis performance in the autofluorescence observation mode.

In the autofluorescence observation mode, repeated image acquisition in units of two frames is not essential. Nor is display of the autofluorescence image in pseudo color essential. In pseudo color display of the autofluorescence image, which color channel of image signal is to be assigned to which color channel may be arbitrarily determined. The autofluorescent substances are not limited to porphyrin and FAD.

In the endoscopic diagnosis system 10A, the image signals of the autofluorescence image are corrected based on the image signal ratio R2/G2. The excitation light and the autofluorescence have different wavelength bands, and the hemoglobin absorption coefficient differs significantly between a central wavelength of 405 nm of the excitation light and a peak emission wavelength of 630 nm of the autofluorescence. Thus, dealing with the absorption by blood of the excitation light and the absorption by blood of the autofluorescence enables improvement in accuracy with which the attenuation of the autofluorescence is corrected in relation to the light absorption by blood.

Next, described below is an embodiment in which attenuation ratios of excitation light and autofluorescence due to absorption by blood are separately obtained, and in which the autofluorescence image is corrected based on the obtained attenuation ratio.

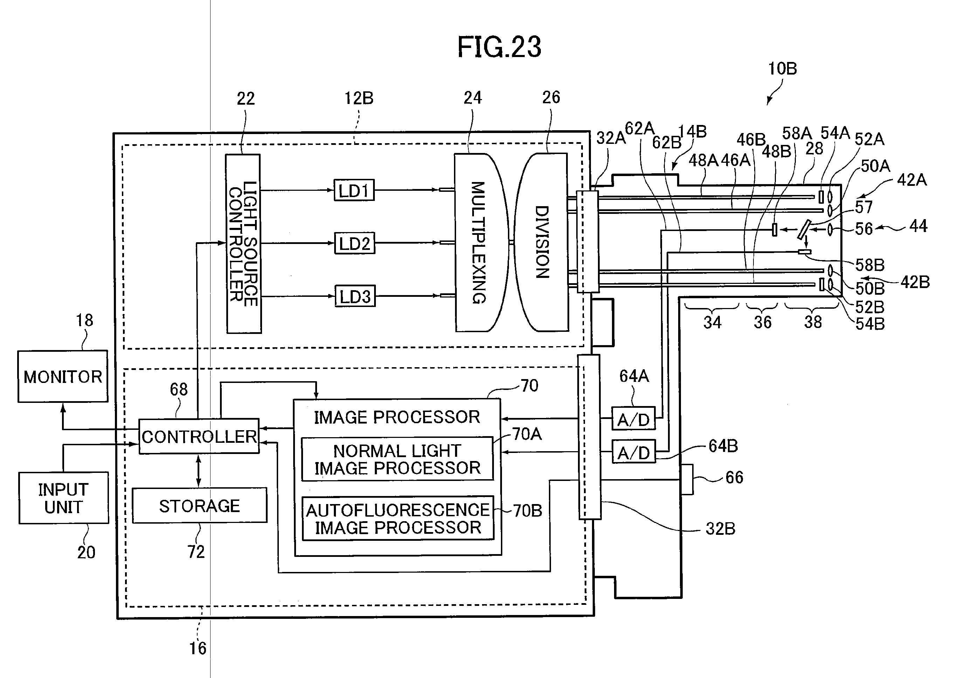

Fig. 23 is a block diagram of the second embodiment illustrating an internal configuration of the endoscopic diagnosis system according to the second aspect shown in Fig. 19. An endoscopic diagnosis system 10B shown in that figure differs from the endoscopic diagnosis system 10A shown in Fig. 20 only in part of the configuration of the light source device, 12B, and the endoscope device, 14B, and like components other than these components are assigned like reference characteristics. No detailed description is made of the shared components below. The description to follow is focused on the differences between the two systems.

As compared with the light source device 12A of the endoscopic diagnosis system 10A shown in Fig. 20, the light source device 12B additionally comprises a laser light source LD3. The laser light source LD3 emits monochromatic light (narrowband light) having a central wavelength of 630 nm and a given wavelength range. The laser light source LD3 emits monochromatic light having the same wavelength band as the peak emission wavelength of the autofluorescence emitted from an autofluorescent substance (porphyrin in this embodiment) that is excited by the laser light source LD1. The light source device 12B is not limited to a light source device that emits single monochromatic light and may emit at least one kind of monochromatic light.

The light source controller 22 turns off the laser light sources LD1, LD3 and turns on the laser light source LD2 in the normal light observation mode. The light source controller 22 sequentially turns on the laser light sources LD1, LD2, and LD3 in the autofluorescence observation mode.

As compared with the endoscope device 14A of the endoscopic diagnosis system 10A shown in Fig. 20, the endoscope device 14B has a half mirror 57 and image sensors 58A, 58B in lieu of the image sensor 58, and A/D converters 64A, 64B in lieu of the A/D converter 64.

The half mirror 57 is provided on the inside of the lens 56 attached at the observation window 44. There are provided image sensors 58A, 58B further along the optical path of the transmitted light through the half mirror 57 and the optical path of the reflected light reflected by the half mirror 57, respectively. The image sensor 58A, a normal sensor, is used in normal light observation (for acquiring the normal light image and a monochromatic light image described later); the image sensor 58B, a high-sensitivity sensor, is used in autofluorescence observation (for acquiring the autofluorescence image). Since the signal intensity or fluorescence intensity of autofluorescence is weak, the image sensor 58B used in this embodiment for autofluorescence observation has a higher sensitivity than the image sensor 58A used for normal light observation.

The image sensors 58A, 58B receive light (transmitted light, reflected light) from the lens 56 at their light receiving surfaces and photoelectrically convert the received light before outputting an imaging signal, an analog signal. The image sensors 58A, 58B have sets of pixels arranged in matrix, one set of pixels being composed of pixels of three colors, an R pixel, a G pixel, and a B pixel. The image sensor 58A is provided on its light receiving surface (optical path) with R, G, and B color filters corresponding to the R pixels, the G pixels, and the B pixels, having a spectral transmission characteristic whereby the reflected light in a wavelength range of about 370 nm to 720 nm of the visible light arriving from the observation region is divided into three for transmission. The image sensor 58B is provided on its light receiving surface (optical path) with R, G, and B color filters corresponding to the R pixels, the G pixels, and the B pixels, having a spectral transmission characteristic of halving and transmitting red and green autofluorescence in a wavelength range of about 500 nm to 700 nm emitted from an autofluorescent substance, while blocking the excitation light having a central wavelength of 405 nm. Thus, the color filters of the image sensor 58B also serve as excitation light cut filters for cutting off light having a central wavelength of, for example, 410 nm or less in order to block excitation light having a central wavelength of 405 nm in the autofluorescence observation mode.

The light emitted from the light source device 12B and guided through the optical fibers 46A, 46B and 48A, 48B is emitted from the endoscope tip portion 38 toward the subject's observation region. The reflected light from an illuminated observation region or autofluorescence emitted from an autofluorescent substance in an observation region is caused by the lens 56 to form an image on the light receiving surfaces of the image sensors 58A, 58B and undergoes photoelectric conversion through the image sensors 58A, 58B for imaging. The image sensors 58A, 58B output imaging signals (analog signals) of the subject's imaged observation region.

In the normal light observation mode, the excitation light for the normal light observation emitted from the light source LD2 is guided through the optical fibers 48A, 48B to irradiate the fluorescent bodies 54A, 54B, whereupon the white light generated from the fluorescent bodies 54A, 54B is emitted from the illumination windows 42A, 42B to illuminate the subject's observation region. The reflected light from the subject's observation region illuminated with the white light is condensed by the lens 56, and dispersed by the color filters, whereupon the normal light image is acquired by the image sensor 58A.

In the autofluorescence observation mode, the excitation light for the autofluorescence observation emitted from the laser light source LD1 is guided through the optical fibers 46A, 46B and emitted from the endoscope tip portion 38 toward the subject's observation region. The autofluorescence emitted from the autofluorescent substance in the subject's observation region irradiated with the excitation light is condensed by the lens 56 and dispersed by the color filters while the excitation light is blocked, whereupon the autofluorescence images are acquired by the image sensor 58B.

The operation whereby excitation light for normal light observation is emitted from the laser light source LD2 in the autofluorescence observation mode is the same as in the normal light observation mode.

The monochromatic light having a central wavelength of 630 nm emitted from the laser light source LD3 in the autofluorescence observation mode is guided through optical fibers 48A, 48B and directed to illuminate the fluorescent bodies 54A, 54B. The monochromatic light having a central wavelength of 630 nm passes through the fluorescent bodies 54A, 54B without exciting the fluorescent bodies 54A, 54B and is directed to illuminate the subject's observation region from the endoscope tip portion 38. The reflected light from the subject's observation region illuminated with the monochromatic light is condensed by the lens 56 and dispersed by the color filters, whereupon the monochromatic light image is acquired by the image sensor 58A.

In the description to follow, R1 and G1 respectively denote the red and the green image signal of the autofluorescence image; R2, G2, and B2 denote the red, the green, and the blue image signal of the normal light image, respectively; and R3 denotes the red image signal of the monochromatic light image.

The monochromatic light having a central wavelength of 630 nm may be guided through the optical fibers 46A, 46B. This embodiment uses an image signal ratio R3/G2 of the red image signal R3 of the monochromatic light image to the green image signal G2 of the normal light image in order to eliminate the effects of light absorption by blood from the autofluorescence image as described later. Therefore, the monochromatic light having a central wavelength of 630 nm is preferably guided through the same optical fibers 48A, 48B as for the normal light image so that the image signal G2 and the image signal G3 are obtained under the same imaging conditions.

Imaging signals (analog signals) of the images (normal light image and autofluorescence image) outputted from the image sensors 58A, 58B are delivered through scope cables 62A, 62B to enter A/D converters 64A, 64B. The A/D converters 64A, 64B convert the imaging signals (analog signals) supplied from the image sensors 58A, 58B to image signals (digital signals). The image signals obtained through the conversion pass through the connector unit 32B to enter the image processor 70 of the processor 16.

The description to follow explains a method of eliminating the effects of light absorption by blood from the autofluorescence image.

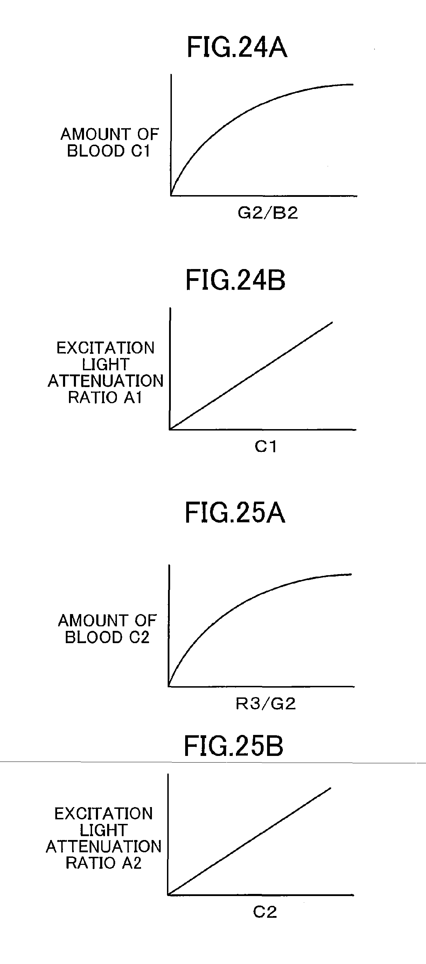

Fig. 24A is a graph of a third conversion table representing a relation between amount of blood and image signal ratio G2/B2 of the green to the blue image signal of the normal light image. In the graph, the vertical axis shows amount of blood C1 corresponding to the excitation light; the horizontal axis shows image signal ratio G2/B2. Fig. 24B is a graph of a fourth conversion table representing a relation between amount of blood C1 and excitation light attenuation ratio A1. In the graph, the vertical axis shows excitation light attenuation ratio A1; the horizontal axis shows amount of blood C1.

Fig. 24, a similar graph to that shown in Fig. 21, shows that the effects of excitation light absorption by blood (amount of blood C1) can be obtained using the image signal ratio G2/B2 in a wavelength band near the excitation light's central wavelength 405 nm, and the ratio A1 of the blood-caused excitation light attenuation can be obtained. Figs. 24A and 24B may be combined into a single graph.

Fig. 25A is a graph of a fifth conversion table representing a relation between amount of blood and image signal ratio R3/G2 of the red image signal of the monochromatic light image to the green image signal of the normal light image. In the graph, the vertical axis shows amount of blood C2 corresponding to the autofluorescence; the horizontal axis shows image signal ratio R3/G2. Fig. 25B is a graph of a sixth conversion table representing a relation between amount of blood C2 and fluorescence attenuation ratio A2. In the graph, the vertical axis shows fluorescence attenuation ratio A2; the horizontal axis shows amount of blood C2.

Fig. 25, also a similar graph to that shown in Fig. 21, shows that the effects of autofluorescence light absorption by blood (amount of blood C2) can be obtained using the image signal ratio R3/G2 near the autofluorescence's peak emission wavelength 630 nm, and then the ratio A2 of the blood-caused excitation light attenuation can be obtained. Figs. 25A and 25B may be combined into a single graph.

From the graphs shown in Fig. 24, the amount of blood C1 corresponding to the image signal ratio G2/B2 is obtained, and the excitation light attenuation ratio A1 corresponding to the obtained amount of blood C1 is obtained. From the graphs shown in Fig. 25, the amount of blood C2 corresponding to the image signal ratio R3/G2 is obtained, and the excitation light attenuation ratio A2 corresponding to the obtained amount of blood C2 is obtained. The excitation light attenuation ratio A1 and the fluorescence attenuation ratio A2 obtained are multiplied to obtain the overall attenuation ratio A3, which is used to correct the red image signal R1 of the autofluorescence image according to the formula (2) given below to obtain the red image signal R1' of the corrected autofluorescence image.

$$R1' = R1/(1 - A3) \qquad (2)$$

Thus, the effects of excitation light absorption by blood and the effects of autofluorescence absorption by blood can be accurately obtained independently, so that the accuracy of correction of the red image signal R1 of the autofluorescence image can be further improved.

Next, the operation of the endoscopic diagnosis system 10B will be described.

In the normal light observation mode, the light source controller 22 controls the laser light sources LD1, LD3 to be turned off and the laser light source LD2 to be turned on. The laser beam having a central wavelength of 445 nm emitted from the laser light source LD2 is directed to irradiate the fluorescent bodies 54A, 54B, causing the fluorescent bodies 54A, 54B to emit white light. The white light emitted from the fluorescent bodies 54A, 54B is directed to illuminate the subject, the reflected light is received by the image sensor 58A, and the normal light image is thereby acquired. The normal light image is displayed in color based on the blue, green, and red image signals (normal light image processing).

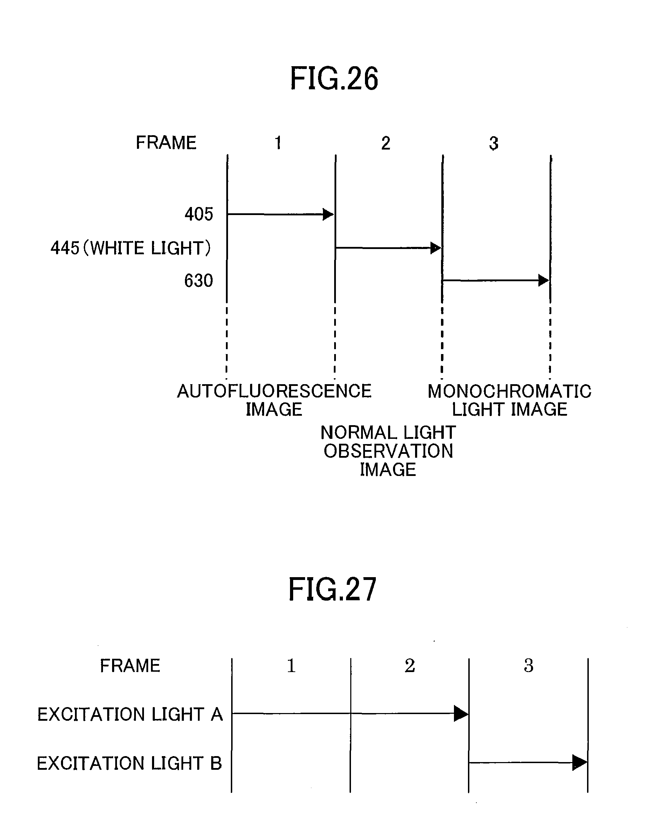

In the autofluorescence observation mode, acquisition of images is performed repeatedly in units of, for example, three frames as illustrated in Fig. 26. In the first of the three frames, the autofluorescence image is acquired; in the second frame, the normal light image is acquired; in the third frame, the monochromatic light image is acquired.

In the first frame, the light source controller 22 controls the laser light source LD1 to be turned on and the laser light sources LD2 and LD3 to be turned off. The laser beam having a central wavelength of 405 nm emitted from the laser light source LD1 is directed to irradiate the subject, whereupon the autofluorescence emitted from the subject is received by the image sensor 58B while the excitation light is cut off, acquiring the autofluorescence image. The autofluorescence image is stored in the storage 72 under the control of the controller 68.

In the first frame, the light source controller 22 controls the laser light source LD2 to be turned on and the laser light sources LD1, LD3 to be turned off to acquire the normal light image as in the normal light observation mode. The normal light image is stored in the storage 72 under the control of the controller 68.

In the third frame, the light source controller 22 controls the laser light source LD3 to be turned on and the laser light sources LD1, LD2 to be turned off. The laser beam having a central wavelength of 630 nm emitted from the laser light source LD3 pass through the fluorescent bodies 54A, 54B to illuminate the subject, whereupon the reflected light thereof is received by the image sensor 58A to acquire the monochromatic light image. The monochromatic light image is stored in the storage 72 under the control of the controller 68.

Subsequently, the autofluorescence image processor 70B uses the autofluorescence image signals and the normal light image signals, and the monochromatic light image signals stored in the storage 72 to obtain the amount of blood C1 corresponding to the image signal ratio G2/B2 and obtain the excitation light attenuation ratio A1 corresponding to the obtained amount of blood C1 from the graphs shown in Fig. 24. From the graphs shown in Fig. 25, the amount of blood C2 corresponding to the image signal ratio R3/G2 is obtained, and the autofluorescence attenuation ratio A2 corresponding to the obtained amount of blood C2 is obtained. The excitation light attenuation ratio A1 and the fluorescence attenuation ratio A2 obtained are multiplied to obtain the overall attenuation ratio A3, and the red image signal R1 of the autofluorescence image is corrected according to the above formula (2). Thus, the effects of light absorption by blood can be eliminated from the red autofluorescence image signal R1. This embodiment shares the procedure to follow with the first embodiment.

Thus, by calculating the amounts of blood separately with respect to the excitation light and the autofluorescence and correcting the red image signal R1 of the autofluorescence, the correction accuracy can be further improved over the first embodiment, and the diagnosis performance in the autofluorescence observation mode can be improved.

In the first embodiment of the endoscopic diagnosis system 10A, correction of the red imaging signal R1 of the autofluorescence image may also be achieved as in the operation of the endoscopic diagnosis system 10B according to the second embodiment by using, for example, the image signal of a first spectral image corresponding to a wavelength of 445 nm and the image signal of a second spectral image corresponding to a wavelength of 630 nm obtained by spectrum estimation method from the image signals of the normal light image acquired in the second frame in the autofluorescence observation mode.

The first spectral image has a central wavelength at 445 nm, at which blue peak wavelength of the normal light image (corresponding to the blue image signal B2 of the normal light image); the second spectral image has a central wavelength at 630 nm, which is a red peak emission wavelength of the autofluorescence (corresponding to the red image signal R3 of the monochromatic light image)

The spectrum estimation method used in the endoscopic diagnosis system 10A according to the first embodiment is not limited in any manner and may selected from a variety of spectrum estimation methods including those known in the art. One may use, for example, a method described in JP 2003-93336 A for spectrum estimation in the present invention, which uses a given coefficient (matrix) obtained based on the spectral characteristic of the illumination light source used and the reflection characteristic of a portion of interest in the subject to generate a spectral image signal corresponding to a given wavelength from the red, the green, and the blue color image signal of the normal light image.

The present invention is basically as described above.

While the invention has been described above in detail, the invention is by no means limited to the above embodiments, and various improvements and modifications may of course be made without departing from the spirit of the present invention.

Preferred Embodiments our outlined in the following paragraphs

1. An endoscopic diagnosis system comprising:

a light source unit for emitting illumination light including at least green and red light or blue and green light or white light, and one or more kinds of excitation light having different central wavelengths for causing at least one autofluorescent substance contained in a subject's observation region to emit one or more kinds of autofluorescence;

an imaging unit for receiving reflected light of the illumination light from the subject's observation region to acquire a reflected light image when the subject's observation region is illuminated with the illumination light emitted from the light source unit, and receiving autofluorescence that is emitted from the at least one autofluorescent substance contained in the subject's observation region, absorbed by blood, and decreases according to an amount of the blood to acquire an autofluorescence image when the subject's observation region is irradiated with the excitation light emitted from the light source unit; and

an image correction unit for correcting the autofluorescence image based on the reflected light image.

2. The endoscopic diagnosis system according to para 1,
wherein the light source unit is adapted to emit the white light as the illumination light and first excitation light as the excitation light,
wherein the image correction unit has correction coefficients for correcting a signal intensity of the autofluorescence image for decrease occurring in accordance with the amount of the blood as the autofluorescence emitted from the at least one autofluorescent substance contained in the subject's observation region is absorbed by the blood, and
wherein the image correction unit is adapted to obtain a correction coefficient corresponding to a reflectance of the reflected light image from among correction coefficients and use the obtained correction coefficient to correct the signal intensity of the autofluorescence image.

3. The endoscopic diagnosis system according to para 2,
wherein the image correction unit is adapted to further correct non-linearity occurring between the reflectance of the reflected light image and the signal intensity of the autofluorescence image according to a difference between a first propagation length over which the reflected light of the white light, occurring when the white light hits the subject's observation region, travels from the subject's observation region until the reflected light is received by the imaging unit and a second propagation length over which the autofluorescence emitted from the at least one autofluorescent substance contained in the subject's observation region when the first excitation light hits the subject's observation region travels until the autofluorescence is received by the imaging unit.

4. The endoscopic diagnosis system according to para 2 or 3,
wherein the image correction unit comprises a correction coefficient table storing relations between a reflectance of the reflected light image and the correction coefficients, and

wherein the image correction unit is adapted to use the correction coefficient table to obtain the correction coefficient corresponding to the reflectance of the reflected light image.

5. The endoscopic diagnosis system according to any one of paras 2 to 4,
wherein the light source unit is adapted to emit excitation light having a given wavelength range with at least one of central wavelengths of 405 nm and 445 nm as the first excitation light.

6. The endoscopic diagnosis system according to para 5,
wherein the light source unit comprises a laser light source that emits the first excitation light.

7. The endoscopic diagnosis system according to any one of paras 2 to 6,
wherein the light source unit comprises a white light source for emitting second excitation light having a given wavelength range with a central wavelength of 445 nm; and a fluorescent body that emits pseudo white light having a given wavelength range containing the second excitation light upon the white light source emitting the second excitation light.

8. The endoscopic diagnosis system according to para 7,
wherein the light source unit comprises a laser light source that emits the second excitation light.

9. The endoscopic diagnosis system according to any one of paras 2 to 8,
wherein the imaging unit comprises an image sensor for acquiring the reflected light image and the autofluorescence image; and a wavelength selection member located on an optical path of the image sensor and having a spectral transmission characteristic of transmitting light having wavelength ranges corresponding to green and red.

10. The endoscopic diagnosis system according to para 9,
wherein the wavelength selection member is color filters.

11. The endoscopic diagnosis system according to para 9,
wherein the wavelength selection member is an etalon, the wavelength range corresponding to the green is 500 nm to 600 nm, and the wavelength range corresponding to the red is 610 nm to 650 nm.

12. The endoscopic diagnosis system according to any one of paras 2 to 8,
wherein the imaging unit comprises a first image sensor for acquiring the reflected light image; a first wavelength selection member located on an optical path of the first image sensor and having a spectral transmission characteristic of transmitting light having wavelengths corresponding to blue, green and red; a second image sensor having a higher sensitivity than the first image sensor for acquiring the autofluorescence image; and a second wavelength selection member located on an optical path of the second image sensor and having a spectral transmission characteristic of transmitting light having wavelengths corresponding to green and red.

13. The endoscopic diagnosis system according to para 12,
wherein the first and the second wavelength selection members are color filters.

14. The endoscopic diagnosis system according to para 12,
wherein the first and the second wavelength selection members are etalons, the wavelength range corresponding to the green is 500 nm to 600 nm, and the wavelength range corresponding to the red is 610 nm to 650 nm.

15. The endoscopic diagnosis system according to any one of paras 9 to 14,
wherein the image correction unit is adapted to correct signal intensities of green and red image signals of the autofluorescence image based on reflectances of green and red image signals of the reflected light image, respectively.

16. The endoscopic diagnosis system according to para 15,
further comprising a monitor for assigning a green image signal of the corrected autofluorescence image to a green channel, and a red image signal to a red channel and a blue channel, and displaying in pseudo color.

17. The endoscopic diagnosis system according to para 1,
wherein the light source unit is adapted to emit the at least green and red light or blue and green light as the illumination light, and the excitation light,

wherein the image correction unit contains information on an attenuation ratio with which the excitation light and the autofluorescence emitted from the at least one autofluorescent substance contained in the subject's observation region decrease by absorption by the blood according to the amount of the blood, and

wherein the image correction unit is adapted to obtain an attenuation ratio corresponding to the image signals of the reflected light image from the information on the attenuation ratio corresponding to the amount of the blood, and use the obtained attenuation ratio to correct the image signals of the autofluorescence image.

18. The endoscopic diagnosis system according to para 17,
wherein the imaging unit is adapted to acquire the reflected light image and the autofluorescence image in turn by frame.

19. The endoscopic diagnosis system according to para 17 or 18,
wherein the imaging unit comprises an image sensor for acquiring the reflected light image and the autofluorescence image; and color filters located on an optical path of the image sensor and having a spectral transmission characteristic of transmitting light having wavelengths corresponding to blue, green and red and not transmitting light having a wavelength range of the excitation light.

20. The endoscopic diagnosis system according to para 19,
wherein the image correction unit is adapted to obtain the attenuation ratio based on an image signal ratio of the red to the green image signal of the reflected light image.

21. The endoscopic diagnosis system according to para 20,
wherein the image correction unit comprises a first conversion table representing a relation between the image signal ratio of the red to the green image signal of the reflected light image and the amount of the blood; and a second conversion table representing a relation between the amount of the blood and the attenuation ratio, and
wherein the image correction unit is adapted to use the first conversion table to obtain the amount of the blood from the image signal ratio of the red to the green image signal of the reflected light image whereas use the second conversion table to obtain the attenuation ratio from the obtained amount of the blood.

22. The endoscopic diagnosis system according to para 21,
wherein the image correction unit is adapted to use a conversion table that is obtained by combining the first and the second conversion table and which represents a relation between the image signal ratio of the red to the green image signal of the reflected light image and the attenuation ratio to obtain the attenuation ratio from the image signal ratio of the red to the green image signal of the reflected light image.

23. The endoscopic diagnosis system according to any one of paras 17 to 22,
wherein the excitation light contains light having a central wavelength of 405 nm, and the autofluorescent substance is porphyrin.

24. The endoscopic diagnosis system according to para 17,
wherein the light source unit is adapted to further emit one or more kinds of monochromatic light having a central wavelength corresponding to a peak emission wavelength of the autofluorescence, and
wherein the imaging unit is adapted to further receive reflected light of monochromatic light from the subject's observation region to acquire a monochromatic light image when the subject's observation region is illuminated with the monochromatic light emitted from the light source unit, and
wherein the imaging unit is adapted to acquire the reflected light image, the autofluorescence image and the monochromatic light image in turn by frame.

25. The endoscopic diagnosis system according to para 24,
wherein the imaging unit comprises a first image sensor for acquiring the reflected light image and the monochromatic light image; color filters located on an optical path of the first image sensor and transmitting light having wavelength ranges corresponding to blue, green and red; a second image sensor having a higher sensitivity than the first image sensor for acquiring the autofluorescence image; and color filters located on an optical path of the second image sensor and having a spectral transmission characteristic of transmitting light having wavelength ranges corresponding to green and red and not transmitting light having a wavelength range of the excitation light.

26. The endoscopic diagnosis system according to para 25,
wherein the image correction unit is adapted to obtain an attenuation ratio of the excitation light based on the image signal ratio of the green to the blue image signal of the reflected light image, obtain the attenuation ratio of the autofluorescence based on an image signal ratio of the red image signal of the monochromatic light image to the green image signal of the reflected light image, and obtain an overall attenuation ratio by multiplying the attenuation ratio of the excitation light and the attenuation ratio of the autofluorescence to correct the image signals of the autofluorescence image.

27. The endoscopic diagnosis system according to para 26,
wherein the image correction unit comprises a third conversion table representing a relation between the image signal ratio of the green to the blue image signal of the reflected light image and an amount of the blood corresponding to the excitation light, a fourth conversion table representing a relation between the amount of the blood corresponding to the excitation light and the attenuation ratio of the excitation light, a fifth conversion table representing a relation between the image signal ratio of the red image signal of the monochromatic light image to the green image signal of the reflected light image and an amount of the blood corresponding to the autofluorescence, and a sixth conversion table representing a relation between the amount of the blood corresponding to the autofluorescence and the attenuation ratio of the autofluorescence, and
wherein the image correction unit is adapted to use the third conversion table to obtain the amount of the blood corresponding to the excitation light from the image signal ratio of the green to the blue image signal of the reflected light image, use the fourth conversion table to obtain the attenuation ratio of the excitation light from the amount of the blood corresponding to the excitation light, use the fifth conversion table to obtain the amount of the blood corresponding to the autofluorescence from the image signal ratio of the red image signal of the monochromatic light image to the green image signal of the reflected light image, and use the sixth conversion table to obtain the attenuation ratio of the autofluorescence from the amount of the blood corresponding to the autofluorescence.

28. The endoscopic diagnosis system according to para 27,
wherein the image correction unit is adapted to use a conversion table obtained by combining the third and the fourth conversion table and representing a relation between the image signal ratio of the green to the blue image signal of the reflected light image and the attenuation ratio of the excitation light to obtain the attenuation ratio of the excitation light from the image signal ratio of the green to the blue image signal of the reflected light image, and use a conversion table obtained by combining the fifth and the sixth conversion table and representing a relation between the image signal ratio of the red image signal of the monochromatic light image to the green image signal of the reflected light image and the attenuation ratio of the autofluorescence to obtain the attenuation ratio of the autofluorescence from the image signal ratio of the red image signal of the monochromatic light image to the green image signal of the reflected light image.

29. The endoscopic diagnosis system according to any one of paras 24 to 28,
wherein the excitation light contains light having a central wavelength of 405 nm, the monochromatic light contains light having a central wavelength of 630 mm, and the autofluorescent substance is porphyrin.

30. The endoscopic diagnosis system according to para 17,
wherein the image correction unit is adapted to further perform spectrum estimation from the image signals of the reflected light image to obtain an image signal of a first spectral image whose central wavelength is a peak wavelength of blue light of the reflected light image, and an image signal of a second spectral image whose central wavelength is a peak emission wavelength of the autofluorescence and obtain an attenuation ratio corresponding to the image signals of the reflected light image and image signals of the first and the second spectral image, and
wherein the imaging unit is adapted to acquire the reflected light image and the autofluorescence image in turn by frame.

31. The endoscopic diagnosis system according to para 30,
wherein the excitation light contains light having a central wavelength of 405 nm, and the autofluorescent substance is porphyrin.

32. The endoscopic diagnosis system according to any one of paras 17 to 31,
wherein the illumination light is white light.

**Claims**

1. An endoscopic diagnosis system comprising:

a light source unit for emitting illumination light including at least green and red light or blue and green light or white light, and one or more kinds of excitation light having different central wavelengths for causing at least one autofluorescent substance contained in a subject's observation region to emit one or more kinds of autofluorescence;

an imaging unit for receiving reflected light of the illumination light from the subject's observation region to acquire a reflected light image when the subject's observation region is illuminated with the illumination light emitted from the light source unit, and receiving autofluorescence that is emitted from the at least one autofluorescent substance contained in the subject's observation region, absorbed by blood, and decreases according to an amount of the blood to acquire an autofluorescence image when the subject's observation region is irradiated with the excitation light emitted from the light source unit; and

an image correction unit for correcting the autofluorescence image based on the reflected light image.

2.  The endoscopic diagnosis system according to Claim 1,
    wherein the light source unit is adapted to emit the white light as the illumination light and first excitation light as the excitation light,
    wherein the image correction unit has correction coefficients for correcting a signal intensity of the autofluorescence image for decrease occurring in accordance with the amount of the blood as the autofluorescence emitted from the at least one autofluorescent substance contained in the subject's observation region is absorbed by the blood, and
    wherein the image correction unit is adapted to obtain a correction coefficient corresponding to a reflectance of the reflected light image from among correction coefficients and use the obtained correction coefficient to correct the signal intensity of the autofluorescence image.

3.  The endoscopic diagnosis system according to Claim 2,
    wherein the image correction unit is adapted to further correct non-linearity occurring between the reflectance of the reflected light image and the signal intensity of the autofluorescence image according to a difference between a first propagation length over which the reflected light of the white light, occurring when the white light hits the subject's observation region, travels from the subject's observation region until the reflected light is received by the imaging unit and a second propagation length over which the autofluorescence emitted from the at least one autofluorescent substance contained in the subject's observation region when the first excitation light hits the subject's observation region travels until the autofluorescence is received by the imaging unit.

4.  The endoscopic diagnosis system according to Claim 2 or 3,
    wherein the image correction unit comprises a correction coefficient table storing relations between a reflectance of the reflected light image and the correction coefficients, and
    wherein the image correction unit is adapted to use the correction coefficient table to obtain the correction coefficient corresponding to the reflectance of the reflected light image.

5.  The endoscopic diagnosis system according to any one of Claims 2 to 4,
    wherein the imaging unit comprises a first image sensor for acquiring the reflected light image; a first wavelength selection member located on an optical path of the first image sensor and having a spectral transmission characteristic of transmitting light having wavelengths corresponding to blue, green and red; a second image sensor having a higher sensitivity than the first image sensor for acquiring the autofluorescence image; and a second wavelength selection member located on an optical path of the second image sensor and having a spectral transmission characteristic of transmitting light having wavelengths corresponding to green and red.

6.  The endoscopic diagnosis system according to Claim 5,
    wherein the image correction unit is adapted to correct signal intensities of green and red image signals of the autofluorescence image based on reflectances of green and red image signals of the reflected light image, respectively.

7.  The endoscopic diagnosis system according to Claim 1,
    wherein the light source unit is adapted to emit the at least green and red light or blue and green light as the illumination light, and the excitation light,
    wherein the image correction unit contains information on an attenuation ratio with which the excitation light and the autofluorescence emitted from the at least one autofluorescent substance contained in the subject's observation region decrease by absorption by the blood according to the amount of the blood, and
    wherein the image correction unit is adapted to obtain an attenuation ratio corresponding to the image signals of the reflected light image from the information on the attenuation ratio corresponding to the amount of the blood, and use the obtained attenuation ratio to correct the image signals of the autofluorescence image.

8. The endoscopic diagnosis system according to Claim 7,
wherein the imaging unit is adapted to acquire the reflected light image and the autofluorescence image in turn by frame.

9. The endoscopic diagnosis system according to Claim 7 or 8,
wherein the imaging unit comprises an image sensor for acquiring the reflected light image and the autofluorescence image; and color filters located on an optical path of the image sensor and having a spectral transmission characteristic of transmitting light having wavelengths corresponding to blue, green and red and not transmitting light having a wavelength range of the excitation light.

10. The endoscopic diagnosis system according to Claim 9,
wherein the image correction unit is adapted to obtain the attenuation ratio based on an image signal ratio of the red to the green image signal of the reflected light image.

11. The endoscopic diagnosis system according to Claim 10,
wherein the image correction unit comprises a first conversion table representing a relation between the image signal ratio of the red to the green image signal of the reflected light image and the amount of the blood; and a second conversion table representing a relation between the amount of the blood and the attenuation ratio, and wherein the image correction unit is adapted to use the first conversion table to obtain the amount of the blood from the image signal ratio of the red to the green image signal of the reflected light image whereas use the second conversion table to obtain the attenuation ratio from the obtained amount of the blood.

12. The endoscopic diagnosis system according to Claim 11,
wherein the image correction unit is adapted to use a conversion table that is obtained by combining the first and the second conversion table and which represents a relation between the image signal ratio of the red to the green image signal of the reflected light image and the attenuation ratio to obtain the attenuation ratio from the image signal ratio of the red to the green image signal of the reflected light image.

13. The endoscopic diagnosis system according to Claim 7,
wherein the light source unit is adapted to further emit one or more kinds of monochromatic light having a central wavelength corresponding to a peak emission wavelength of the autofluorescence, and
wherein the imaging unit is adapted to further receive reflected light of monochromatic light from the subject's observation region to acquire a monochromatic light image when the subject's observation region is illuminated with the monochromatic light emitted from the light source unit, and
wherein the imaging unit is adapted to acquire the reflected light image, the autofluorescence image and the monochromatic light image in turn by frame.

14. The endoscopic diagnosis system according to Claim 13,
wherein the imaging unit comprises a first image sensor for acquiring the reflected light image and the monochromatic light image; color filters located on an optical path of the first image sensor and transmitting light having wavelength ranges corresponding to blue, green and red; a second image sensor having a higher sensitivity than the first image sensor for acquiring the autofluorescence image; and color filters located on an optical path of the second image sensor and having a spectral transmission characteristic of transmitting light having wavelength ranges corresponding to green and red and not transmitting light having a wavelength range of the excitation light.

15. The endoscopic diagnosis system according to Claim 14,
wherein the image correction unit is adapted to obtain an attenuation ratio of the excitation light based on the image signal ratio of the green to the blue image signal of the reflected light image, obtain the attenuation ratio of the autofluorescence based on an image signal ratio of the red image signal of the monochromatic light image to the green image signal of the reflected light image, and obtain an overall attenuation ratio by multiplying the attenuation ratio of the excitation light and the attenuation ratio of the autofluorescence to correct the image signals of the autofluorescence image.

16. The endoscopic diagnosis system according to Claim 15,
wherein the image correction unit comprises a third conversion table representing a relation between the image signal ratio of the green to the blue image signal of the reflected light image and an amount of the blood corresponding to the excitation light, a fourth conversion table representing a relation between the amount of the blood corresponding to the excitation light and the attenuation ratio of the excitation light, a fifth conversion table representing a relation between the image signal ratio of the red image signal of the monochromatic light image to the green image signal of the reflected light image and an amount of the blood corresponding to the autofluorescence, and a sixth conversion table representing a relation between the amount of the blood corresponding to the autofluorescence and the attenuation ratio of the autofluorescence, and

wherein the image correction unit is adapted to use the third conversion table to obtain the amount of the blood corresponding to the excitation light from the image signal ratio of the green to the blue image signal of the reflected light image, use the fourth conversion table to obtain the attenuation ratio of the excitation light from the amount of the blood corresponding to the excitation light, use the fifth conversion table to obtain the amount of the blood corresponding to the autofluorescence from the image signal ratio of the red image signal of the monochromatic light image to the green image signal of the reflected light image, and use the sixth conversion table to obtain the attenuation ratio of the autofluorescence from the amount of the blood corresponding to the autofluorescence.

17. The endoscopic diagnosis system according to Claim 16,
wherein the image correction unit is adapted to use a conversion table obtained by combining the third and the fourth conversion table and representing a relation between the image signal ratio of the green to the blue image signal of the reflected light image and the attenuation ratio of the excitation light to obtain the attenuation ratio of the excitation light from the image signal ratio of the green to the blue image signal of the reflected light image, and use a conversion table obtained by combining the fifth and the sixth conversion table and representing a relation between the image signal ratio of the red image signal of the monochromatic light image to the green image signal of the reflected light image and the attenuation ratio of the autofluorescence to obtain the attenuation ratio of the autofluorescence from the image signal ratio of the red image signal of the monochromatic light image to the green image signal of the reflected light image.

18. The endoscopic diagnosis system according to Claim 7,
wherein the image correction unit is adapted to further perform spectrum estimation from the image signals of the reflected light image to obtain an image signal of a first spectral image whose central wavelength is a peak wavelength of blue light of the reflected light image, and an image signal of a second spectral image whose central wavelength is a peak emission wavelength of the autofluorescence and obtain an attenuation ratio corresponding to the image signals of the reflected light image and image signals of the first and the second spectral image, and
wherein the imaging unit is adapted to acquire the reflected light image and the autofluorescence image in turn by frame.

# FIG.1

# FIG.2

EP 2 564 760 A1

# FIG.3

# FIG.4

# FIG.5

# FIG.6

## FIG.7

## FIG.8

# FIG.9

NORMALIZED
SIGNAL INTENSITY
[a.u.]

————— FLUORESCENCE INTENSITY

------- REFLECTANCE

1

BLOOD CONCENTRATION

# FIG.10

FLUORESCENCE
INTENSITY
[a.u.]

————— NON-LINEAR

------- LINEAR

REFLECTANCE [a.u.]

# FIG.11

CORRECTION
COEFFICIENT

——————— NON-LINEAR

- - - - - - LINEAR

REFLECTANCE [a.u.]

# FIG.12

FLUORESCENCE
INTENSITY
[a.u.]   405nm LASER BEAM     TRANSMITTANCE

G          R

100

——————— NORMAL
REGION

- - - - - - LESION

WAVELENGTH [nm]

400       500      600      700

# FIG.13

FLUORESCENCE
INTENSITY
[a.u.]   445nm LASER BEAM          TRANSMITTANCE

G    R

——————— NORMAL
            REGION

-------- LESION

WAVELENGTH [nm]

400      500    600    700

# FIG.14

FLUORESCENCE   405nm LASER BEAM
INTENSITY      / 445nm LASER BEAM
[a.u.]        /         TRANSMITTANCE

G    R

100

——————— NORMAL
            REGION

-------- LESION

WAVELENGTH [nm]

400      500    600    700

# FIG.15

# FIG.16

NORMAL LIGHT
OBSERVATION → WHITE LIGHT → SUBJECT → NORMAL SENSOR (R,G,B) → NORMAL LIGHT IMAGE DISPLAYED IN R,G,B COLOR

SPECIAL LIGHT
OBSERVATION
(AUTOFLUORESCENCE
OBSERVATION)

EXCITATION LIGHT 1
EXCITATION LIGHT 2
→ SUBJECT → HIGH SENSITIVITY SENSOR (R,G); CUTS OFF EXCITATION LIGHT IN B CHANNEL → R AND G SIGNALS OF NORMAL SENSOR USED TO CORRECT R AND G SIGNALS OF HIGH SENSITIVITY SENSOR; SIGNALS R' AND G' AFTER CORRECTION → AUTOFLUORESCENCE DISPLAYED IN PSEUDO COLOR: G' ⇒ G; R' ⇒ R,B

EP 2 564 760 A1

# FIG.17

| FRAME | 1 | 2 |

445 (WHITE LIGHT)

405 (MONOCHROMATIC LIGHT)

445 (MONOCHROMATIC LIGHT)

NORMAL LIGHT
OBSERVATION
MODE

AUTOFLUORESCENCE
OBSERVATION
MODE

# FIG.18

| | NORMAL REGION | LESION | ASSIGNMENT |
|---|---|---|---|
| FAD:G-CHANNEL FLUORESCENCE IMAGE WITH 445-nm EXCITATION LIGHT | BRIGHT | DARK G | G |
| PORPHYRIN: R-CHANNEL FLUORESCENCE IMAGE WITH 405-nm EXCITATION LIGHT | DARK G | BRIGHT | R,B |

35

# FIG.19

# FIG.20

# FIG.21A

AMOUNT OF
BLOOD C

R/G

# FIG.21B

ATTENUATION
RATIO A

C

# FIG.22

FRAME     1        2

405

445(WHITE LIGHT)

NORMAL
LIGHT
IMAGE

AUTOFLUORESCENCE
IMAGE

# FIG.23

# FIG.24A

AMOUNT OF
BLOOD C1

G2/B2

# FIG.24B

EXCITATION
LIGHT
ATTENUATION
RATIO A1

C1

# FIG.25A

AMOUNT OF
BLOOD C2

R3/G2

# FIG.25B

EXCITATION
LIGHT
ATTENUATION
RATIO A2

C2

# FIG.26

FRAME 　　　1　　　　　2　　　　　3

405

445 (WHITE LIGHT)

630

AUTOFLUORESCENCE
IMAGE

NORMAL LIGHT
OBSERVATION
IMAGE

MONOCHROMATIC
LIGHT IMAGE

# FIG.27

FRAME 　　　1　　　　　2　　　　　3

EXCITATION LIGHT A

EXCITATION LIGHT B

# FIG.28

SPECIAL LIGHT
OBSERVATION
(AUTOFLUORESCENCE
OBSERVATION)

| EXCITATION LIGHT A | → | SUBJECT | → | IMAGE SIGNAL D1 | → |

| EXCITATION LIGHT A | → | SUBJECT | → | IMAGE SIGNAL D2 | → |

| EXCITATION LIGHT B | → | SUBJECT | → | IMAGE SIGNAL D3 | → |

$D1-D2 \Rightarrow$
SIGNAL E1

AUTOFLUORESCENCE IMAGE
COLOR DISPLAY WITH
$D2 \Rightarrow R, E1 \Rightarrow G, D3 \Rightarrow B$

Europäisches
Patentamt

European
Patent Office

Office européen
des brevets

## EUROPEAN SEARCH REPORT

Application Number

EP 12 17 7855

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 105 082 A1 (OLYMPUS CORP [JP]) 30 September 2009 (2009-09-30) | 1,2,7-18 | INV. A61B1/06 |
| Y | * the whole document * | 3,4 | |
| X | US 5 749 830 A (KANEKO MAMORU [JP] ET AL) 12 May 1998 (1998-05-12) | 1,7-18 | |
| Y | * column 37, line 56 - column 39, line 47; figures 35,32 * <br> * column 34, paragraph 38-60 * | 4 | |
| Y | US 2006/025692 A1 (ISHIHARA YASUSHIGE [JP]) 2 February 2006 (2006-02-02) * paragraphs [0134] - [0138]; figure 1 * | 3 | |
| A | EP 2 108 300 A1 (OLYMPUS CORP [JP]) 14 October 2009 (2009-10-14) * the whole document * | 1-18 | |

TECHNICAL FIELDS
SEARCHED     (IPC)

A61B

The present search report has been drawn up for all claims

| Place of search | Date of completion of the search | Examiner |
|---|---|---|
| Munich | 17 December 2012 | Schindler, Martin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

ANNEX TO THE EUROPEAN SEARCH REPORT
ON EUROPEAN PATENT APPLICATION NO.

EP 12 17 7855

This annex lists the patent family members relating to the patent documents cited in the above-mentioned European search report.
The members are as contained in the European Patent Office EDP file on
The European Patent Office is in no way liable for these particulars which are merely given for the purpose of information.

17-12-2012

| Patent document cited in search report | | Publication date | Patent family member(s) | | Publication date |
|---|---|---|---|---|---|
| EP 2105082 | A1 | 30-09-2009 | CN | 101583304 A | 18-11-2009 |
| | | | EP | 2105082 A1 | 30-09-2009 |
| | | | JP | 5074044 B2 | 14-11-2012 |
| | | | JP | 2008173290 A | 31-07-2008 |
| | | | US | 2010059690 A1 | 11-03-2010 |
| | | | WO | 2008088002 A1 | 24-07-2008 |
| US 5749830 | A | 12-05-1998 | NONE | | |
| US 2006025692 | A1 | 02-02-2006 | JP | 2006061683 A | 09-03-2006 |
| | | | US | 2006025692 A1 | 02-02-2006 |
| EP 2108300 | A1 | 14-10-2009 | EP | 2108300 A1 | 14-10-2009 |
| | | | JP | 2008183349 A | 14-08-2008 |
| | | | US | 2010103250 A1 | 29-04-2010 |
| | | | WO | 2008093746 A1 | 07-08-2008 |

EPO FORM P0459

For more details about this annex : see Official Journal of the European Patent Office, No. 12/82

## REFERENCES CITED IN THE DESCRIPTION

**Patent documents cited in the description**

- JP 2009095683 A

- JP 2008043383 A
- JP 2003093336 A

**Non-patent literature cited in the description**

- Hikari niyoru Igaku Shindan. **MAMORU TAMURA.** Hikariga Hiraku Seimei Kagaku. KYORITSU SHUPPAN CO., LTD, 18 March 2001, vol. 6